# EUROPEAN PATENT SPECIFICATION

(11) **EP 4 121 554 B1**
(45) Date of publication and mention of the grant of the patent: **30.10.2024**
(21) Application number: 21771116.7
(22) Date of filing: 18.03.2021
(51) Int. Cl.: C12Q 1/68

(54) **RESTORING PHASE IN MASSIVELY PARALLEL SEQUENCING**
WIEDERHERSTELLUNG DER PHASE IN MASSIV PARALLELER SEQUENZIERUNG
RESTAURATION DE PHASE DANS UN SÉQUENÇAGE MASSIVEMENT PARALLÈLE

(30) Priority: 18.03.2020 US 202062991440 P
(43) Date of publication of application: 25.01.2023
(73) Proprietor: MGI Tech Co., Ltd., Shenzhen, Guangdong 518083 (CN)
(72) Inventor: CALLOW, Matthew J., San Jose, California 95134 (US); DRMANAC, Radoje, San Jose, California 95134 (US); DRMANAC, Snezana, San Jose, California 95134 (US)
(74) Representative: Patent Boutique LLP
(86) International application number: PCT/CN2021/081575
(87) International publication number: WO 2021/185320

(56) References cited:
- WO-A1-2019/156910
- WO-A1-2021/133685
- WO-A2-2010/127304
- WO-A2-2011/156707
- CN-A- 106 434 866
- US-A1- 2008 293 071
- US-A1- 2017 044 601
- US-A1- 2019 218 599
- US-A1- 2020 032 322
- MENGLING ZHANG;YIHONG HU;XIAOLI ZHU;CHIYU ZHANG: "A method for synthesis of dideoxynucleotide blocked oligonucleotides", BIOTECHNOLOGY, vol. 27, no. 6, 20 December 2017 (2017-12-20), pages 545 - 551, XP055977416, ISSN: 1004-311x, DOI: 10.16519/j.cnki.1004-311x.2017.06.0087
- RIBEIRO BEATRIZ REGALADO GALVAO, MARILIA REGALADO GALVAO RABELO CALDAS, ANTONIO ALVES ALMEIDA JR., RENATA GARCIA FONSECA, GELSON L: "Effect of surface treatments on repair with composite resin of a partially monoclinic phase transformed yttrium-stabilized tetragonal zirconia", THE JOURNAL OF PROSTHETIC DENTISTRY, vol. 119, no. 2, 28 February 2018 (2018-02-28), pages 286 - 291, XP055852160, DOI: 10.1016/j.prosdent.2017.02.014

## Description

### FIELD OF THE INVENTION

The technology in this disclosure relates generally to determining a nucleic acid sequence of a target polynucleotide, such as genomic DNA.

### BACKGROUND

The need for low cost, high-throughput methods for nucleic acid sequencing and re-sequencing has led to the development of "massively parallel sequencing" (MPS) technologies.

One commonly used method for sequencing DNA is referred to as "sequencing-by-synthesis" (SBS), such as disclosed in Ronaghi et al., Science, 281:363-365, 1998; Li et al., Proc. Natl. Acad. Sci. USA, 100:414-419, 2003; Metzker, Nat Rev Genet.11:31-46, 2010; Ju et al., Proc. Natl. Acad. Sci. USA 103:19635-19640, 2006; Bentley et al., Nature 456:53-59, 2008; and in U.S. Pat. Nos. 6,210,891, 6,828,100, 6,833,246, and 6,911,345, and 10,190,162. SBS usually requires extension of a primer hybridized to a single stranded template polynucleotide by the controlled incorporation of the correct complementary nucleotide opposite the template being sequenced. The resulting product is sometimes referred to as the "extended primer" or "growing strand." In one approach, reversible terminator nucleotides (RTs) are used to determine the sequence of the DNA template. In the most commonly used SBS approach, each RT comprises a modified nucleotide that includes: (1) a blocking group that ensures that only a single base can be added by a DNA polymerase enzyme to the 3' end of the growing DNA copy strand, and (2) a fluorescent label. An alternative method, referred to as CoolMPS^{®} sequencing, in which unlabeled reversible terminator nucleotides are used is described in U.S. Patent No. 10,851,410 and in Drmanac, S. et al., bioRxiv 2020.02.19.953307. WO 2019/156910 A1 describes a method of characterizing a nucleic acid that includes steps of (a) contacting a primer-template nucleic acid hybrid with a polymerase and a mixture of nucleotides to produce an extended primer hybrid and to form a stabilized ternary complex including the extended primer hybrid, the polymerase and a nucleotide cognate of the next base in the template, wherein the mixture contains nucleotide cognates of four different base types, wherein the nucleotide cognate of the first base type has a reversible terminator, and wherein nucleotide cognates of the second, third and fourth base types are extendable; (b) detecting the stabilized ternary complex to distinguish the next base from other base types in the template; and (c) determining the presence of a base multiplet in the template nucleic acid, the base multiplet including the first base type followed by the next base.

### SUMMARY OF THE INVENTION

The invention provides a method of rephasing extended primers in a clonal population of nucleic acid duplexes as defined by claim 1. Further features of the invention are defined by the dependent claims. In some massively parallel sequencing (MPS) methods, determining the sequence of a nucleic acid typically entails (i) preparing a library of different template sequences, (ii) immobilizing many copies (a "clonal population") of each template sequence at different sites on a substrate and (iii) performing multiple cycles of a sequencing reaction. The different sites on the substrate may be positioned at random, spaced apart, positions or the sites may be arranged as an ordered array. The sequencing reaction generates a signal at each position on the array. The signal is produced by a process that includes the incorporation of a nucleotide into each of the copies of template. Sequencing a clonal population at each site (rather than a single template molecule) results in a stronger signal, improves accuracy, and, in some methods, reduces amplification errors that occur in the course of producing the clonal population.

However, these advantages require that, in each cycle, incorporation of a complementary nucleotide at each position occurs in phase. That is, in each cycle in which a nucleotide is incorporated the incorporation occur in most or nearly all of the template copies at that position on the array. However, in each cycle there is a chance that non-incorporation or misincorporation will occur for a certain number of the templates of the clonal population at that site. A template for which a non-incorporation or misincorporation may fall out of phase with the other templates at the site. As the number of cycles increases, the out-of-phase templates accumulate at each site, increasing signal-to-noise ratio, compromising accuracy, and limiting read length at the sites. This disclosure describes rephasing strategies for reducing the number of out-of-phase templates, resulting in improvements in accuracy and read length.

Examples of clonal populations of a template sequence include, for illustration and without limitation, DNA nanoballs (single stranded concatemers with many copies of a template sequence along with adaptor sequences that include a primer binding site) including products of in situ amplification of DNBs), double stranded DNA concatemers, clonal clusters of amplicons produced by Bridge Amplification or other amplification methods.

In general terms, this disclosure provides a method of rephasing extended primers in a clonal population of nucleic acid duplexes comprising extended primers hybridized to a template sequence, wherein a plurality of the extended primers in the clonal population have different 3' ends and are thereby out of phase. This can be done by:
(1) extending the extended primers by incorporating one or more nucleotides that are complementary to the template sequence using a polymerase and nucleotides comprising nucleotide triphosphates A, T, C, and G, or analogs thereof, wherein one of the nucleotides is a reversible terminator blocked with a first blocking group and the other three nucleotides are not blocked, until substantially all of the extended primers are blocked; and then
(2) unblocking the extended primers.

This general approach includes the technique of dinucleotide-frequency rephasing (DFR). Each extended primer is extended until a selected dinucleotide is reached, wherein the selected dinucleotide has the formula XY. The first nucleotide of the dinucleotide (X) can be a reversible terminator blocked with the first blocking group, and Y is the second nucleotide of the dinucleotide. The second nucleotide (Y) can be a reversible terminator blocked with a second blocking group, or a blocked degenerage oligonucleotide containing Y, e.g., at the 5 prime termius.

Included in the general DFR approach is a method of rephasing extended primers in a clonal population of nucleic acid duplexes to a selected dinucleotide (XY), wherein the duplexes each comprise an extended primer annealed to a template sequence. The method is beneficial when the extended primers in the clonal population have different 3 prime ends and are thereby out of phase. The rephasing method is done by performing multiple cycles of the following: (i) extending the extended primers using a first mixture that contains a polymerase and four nucleotide triphosphates selected from A, T, C, and G and/or analogs thereof, wherein one of the nucleotide triphosphates or analogs in the first mixture corresponds to the first nucleotide (X) of the selected dinucleotide and is blocked with a first blocking group, and wherein the other three nucleotide triphosphates or analogs in the first mixture are unblocked, the extending being continued until substantially all of the extended primers are blocked with the first blocking group.

Subsequent steps include (ii) unblocking the first blocking group; and (iii) treating the extended primers from (ii) with a second mixture that contains a polymerase and a single nucleotide triphosphate selected from A, T, C, or G and analogs thereof that corresponds to the second nucleotide (Y) of the selected dinucleotide and is blocked with a second blocking group. The second mixture optionally includes the three nucleotide triphosphates or analogs not corresponding to the second nucleotide (Y) blocked with the first blocking group (or, in some embodiments with multiple first blocking groups that do not include the second blocking group), For example, the combination A ◄ T* C** G*** where ◄ is the second blocking group and *, **, and *** are first blocking groups and the second clocking group os not unblocked under the same conditions that unblock blocking group-1.) The multiple cycles are repeated until substantially all of the extended primers are blocked with the second blocking group. Then the second blocking group is unblocked, thereby rephasing the extended primers in the clonal population.

In one such method, the only nucleotide triphosphate in the second mixture is the nucleotide triphosphate or analog that is blocked by the second blocking group. Alternatively, the second mixture contains the nucleotide triphosphate or analog blocked by the second group, and also contains the three nucleotide triphosphates or analogs not corresponding to the second nucleotide (Y) blocked with the first blocking group. Either of the first and second blocking groups may be an O-azidomethyl group, and the other of the first and second blocking groups may be an O-NHz group.

Another variation of the general approach to DFR uses an oligonucleotide to identify the second nucleotide. This is done by performing multiple cycles of the following: (i) extending the extended primers using a first mixture that contains a polymerase and four nucleotide triphosphates selected from A, T, C, and G and/or analogs thereof, wherein one of the nucleotide triphosphates or analogs in the first mixture corresponds to the first nucleotide (X) of the selected dinucleotide and is blocked with a first blocking group, and wherein the other three nucleotide triphosphates or analogs in the first mixture are unblocked, the extending being continued until substantially all of the extended primers are blocked with the first blocking group; then (ii) unblocking the first blocking group; and (iii) treating the extended primers with a second mixture that contains a ligase and a 5' phosphorylated oligonucleotide. The oligonucleotide is blocked at the 3' end, wherein a base in the oligonucleotide corresponds to the second nucleotide (Y) of the selected dinucleotide. The multiple cycles are again repeated until substantially all of the extended primers are blocked with the oligonucleotide; whereupon the oligonucleotide is unblocked, thereby rephasing the extended primers in the clonal population.

The 5' phosphorylated oligonucleotide optionally has the formula 5'⁻phos-B(N)_{Z}-X, where "5'-phos" is a phosphorylated nucleotide, B is an nucleotide that defines a clevage site, X is a blocking structure (i.e., a structure that prevents polymerase mediated extension of the oligonucleotide), which may be a non-reversible blocking structure, Z is 6-20, preferably 6-15, more preferably 6-12, and (N)_{Z} is a degenerate oligonucleotide sequence. In some examples Z is 9. In some examples B is uracil (U). The unblocking may include removing the entire oligonucleotide from the extended primer. Optionally, the non-reversible blocking structure in the oligonculeotide is inverted dT (IDT) incorporated at the 3'-end, thereby creating a 3'-3' linkage which inhibits both degradation by 3' exonucleases and extension by DNA polymerases. When B is uracil, the 5' phosphorylated oligonucleotide can be unblocked by treating with an enzyme mixture of uracil-DNA glycosylase (UDG) and apurinic/apyrimidinic endonuclease 1 (Ape1) to cleave and remove the uracil base. Typically, in any of the dinucleotide phasing methods, five to fifteen cycles are performed.

To avoid creating a gap in the sequence read during rephasing, the user may wish to remove five to fifty bases from the 3' end of each primer before the rephasing, thereby readjusting the 3' end of the extended primers to an upstream position. This can be done in several different ways: for example, during sequencing-by-synthesis done before the rephasing, including in at least some of the cycles of the sequencing a uracil triphosphate or analog thereof that can be incorporated into the extended primer in place of thymine triphosphate; then cleaving the extended primers at incorporated uracil bases. The cleaving may be done using an enzyme mixture of uracil-DNA glycosylase (UDG) and apurinic/apyrimidinic endonuclease 1 (Ape1).

Another way of adjusting the 3' end of the extended primers upstream is as follows: during sequencing-by-synthesis done before the rephasing, including in at least some of the cycles of the sequencing a nucleotide triphosphate that contains an ribonucleotide (RNA) or a 5' alpha-phosphate thio-modified nucleotide; then cleaving the extended primers at incorporated RNA bases or at incorporated 5' alpha-phosphate thio-modified nucleotides. Alternatively, the readjusting comprises treating the extended primers with a 3' exonuclease under controlled conditions, or treating the extended primers with a nicking enzyme that is sub-sequence dependent, thereby removing said five to fifty bases from the 3' end of the extended primer.

After the aforesaid rephasing methods, cycles of sequencing can be resumed, whereby the extended primers in the clonal population are extended by bases that each identify a complementary nucleotide in the template sequence. Long sequencing reads can be obtained from a clonal population of nucleic acid duplexes (each comprising an extended primer annealed to a template sequence) by performing multiple cycles of sequencing in which the extended primer in each duplex is extended by one nucleotide, thereby identifying a complementary nucleotide in the template sequence. After a number of such sequencing cycles, rephasing the extended primers as put forth above, then resuming cycles of the sequencing to identify further nucleotides in the template sequence.

The rephasing is done once, twice, or as often as desired to reduce discordance between primers in a clonal population: typically two to four times within the first 800 sequencing cycles. The benefit of this includes extending the number of clonal populations having a discordance percentage of less than 5%, 2% or 1% by at least 2-fold, 5-fold, or more, or by at least 100, 200, or 400 cycles.

Also provided in this disclosure is a method of sequencing that includes providing an array comprising a plurality of clonal populations, each clonal population comprising nucleic acid duplexes in which a sequencing primer is annealed to a template sequence. Multiple cycles of sequencing-are performed, (for example, by sequencing-by-synthesis) to extend the sequencing primers until at least some of the primers have 3 prime ends that are different from other primers in the same clonal population, and are therefore out of phase with other primers in the clonal population. The sequencing primers are then rephased using any of the methods and optional features put forth above.

Rephasing is done as often as needed or desired during the sequencing process: for example, two to four times during the sequencing, thereby obtaining a read length of at least 800 bases, optionally at least 1200, 1600, or 2000 bases.

A method of rephasing a plurality of copies of a nucleic acid being sequenced, wherein each copy comprises a single stranded nucleic acid template hybridized to an extendible oligonucleotide sequencing probe, the method comprising:
(1) extending the probe in a manner that is complementary to the template using a polymerase and a mixture of nucleotide triphosphates, wherein one of the nucleotide triphosphates is a 3' reversibly blocked nucleotide triphosphate, until substantially all of the copies are blocked; then
(2) unblocking the reversibly blocked nucleotides that are incorporated in the extended probes.

In one aspect provided is a method of rephasing extended sequence primers that are hybridized to a plurality of copies of a nucleic acid template for the purpose of sequencing the template, wherein primers hybridized to at least some of the copies have different '3 ends and are thereby out of phase with primers hybridized to other copies of the template, the method comprising: (1) further extending the primer hybridized to each of the copies by one or more bases that are complementary to the template using a polymerase and a mixture that contains nucleotide triphosphates A, T, C, and G, wherein one of the nucleotide triphosphates is a 3' reversibly blocked nucleotide triphosphate and the other three nucleotide triphosphates are unblocked, until substantially all of the primers are blocked; then (2) unblocking the reversibly blocked nucleotides that are incorporated into the primers.

In one aspect provided is a method of rephasing, which is a method of dinucleotide-frequency rephasing (DFR), in which each primer is extended until a selected dinucleotide is reached. In one aspect provided is a method of dinucleotide-frequency rephasing (DFR) according to claim 2 that comprises: (a) performing multiple cycles of rephasing, wherein each cycle includes the following: further extending the primer hybridized to each of the copies by one or more bases that are complementary to the template using a first mixture that contains a polymerase and nucleotide triphosphates A, T, C, and G, wherein one of the nucleotide triphosphates in the first mixture represents the first nucleotide of the selected dinucleotide and is blocked with a first blocking group, and wherein the other three nucleotide triphosphates in the first mixture are unblocked, the extending being continued until substantially all of the primers are blocked; then unblocking the first blocking group; and treating the primers with a second mixture that contains a polymerase and a single nucleotide triphosphate selected from A, T, C, or G that represents the second nucleotide of the selected dinucleotide and is blocked with a second blocking group, wherein the other three nucleotide triphosphates, if present in the second mixture, are blocked with the first blocking group, wherein the treating results in further extending and blocking only those primers that are adjacent to a base in the template to which the selected nucleotide is complementary; (b) after completing all of the multiple cycles of step (a), unblocking the second blocking group; thereby rephasing the plurality of copies, wherein the 3' end of the primer hybridized to each of the copies is the dinucleotide. In some cases the method of claim 3, wherein the only nucleotide triphosphate in the second mixture is the nucleotide triphosphate that is blocked by the second blocking group. In some cases the method of claim 3, wherein the second mixture contains the nucleotide triphosphate blocked by the second group, along with the other three nucleotide triphosphates blocked with the first blocking group. In some cases the method of claims 3 to 5, wherein the first blocking group is an O azidomethyl or an ONH2 group, and the second blocking group comprises a disulfide bond.

In some cases the nucleotide triphosphate blocked with the first blocking group is C, the nucleotide triphosphate blocked with the second blocking group is A, and the dinucleotide is CA. In some cases the method of dinucleotide-frequency rephasing (DFR) according to claim 2 that comprises: (a) performing multiple cycles of rephasing, wherein each cycle includes the following: further extending the primer hybridized to each of the copies by one or more bases that are complementary to the template using a first mixture that contains a polymerase and nucleotide triphosphates A, T, C, and G, wherein one of the nucleotide triphosphates in the first mixture represents the first nucleotide of the selected dinucleotide and is blocked with a first blocking group, and wherein the other three nucleotide triphosphates in the first mixture are unblocked, the extending being continued until substantially all of the primers are blocked; then unblocking the first blocking group; and treating the primers with a second mixture that contains a ligase and a 5' phosphorylated oligonucleotide blocked at the 3' end with a second blocking group, wherein a base in the oligonucleotide represents the second nucleotide of the selected dinucleotide, and wherein the treating results in ligation of the oligonucleotide only to primers that are adjacent to a portion of the template to which the oligonucleotide is complementary; (b) after completing all the multiple cycles of step (a), unblocking the oligonucleotide. In some cases the method of method of claim 8, wherein the 5' phosphorylated oligonucleotide has the formula BN1 15X, wherein B is a nucleotide base that represents the second nucleotide of the selected dinucleotide, each N is a nucleotide homolog or a nucleotide mixture containing a nucleotide that can hybridize to any base in the template; and X is a non-reversible blocking structure; and wherein the unblocking in step (b) comprises removing the oligonucleotide from the primer.

In some cases the method of method of claim 9, wherein the non-reversible blocking structure is inverted dT (IDT) incorporated at the 3'-end of the oligonucleotide, thereby creating a 3'-3' linkage which inhibits both degradation by 3' exonucleases and extension by DNA polymerases. In some cases the method of method of claim 9, wherein B is uracil, and wherein the 5 phosphorylated oligonucleotide is unblocked by treating with an enzyme mixture of uracil DNA glycosylase (UDG) and apurinic/apyrimidinic endonuclease 1 (Ape1) to cleave and remove the uracil base.

In general terms, the rephasing methods of this disclosure include the following steps: (1) extending the primer in a manner that is complementary to the template using a polymerase and a mixture of nucleotide triphosphates, wherein one of the nucleotide triphosphates is a 3' reversibly blocked nucleotide triphosphate, until substantially all of the copies are blocked; then (2) unblocking the reversibly blocked nucleotides that are incorporated in the extended primers. In its elemental form, the rephasing can be done using a single blocked nucleotide ("Method One", explained in more detail below).

An included variation of this rephasing strategy is dinucleotide-frequency rephasing (DFR) ("Method Two"). Here, the rephasing doesn't occur at a single base, but at a selected nucleotide doublet. The method comprises performing multiple cycles of rephasing, wherein each cycle includes the following: extending the primer in a manner that is complementary to the template using a polymerase and a first mixture of nucleotide triphosphates, wherein one of the nucleotide triphosphates in the first mixture is blocked with a first blocking group, until substantially all of the copies are blocked. The first blocking group is then unblocked, and the primer is extended by a single nucleotide (the second nucleotide of the doublet) in a manner that is complementary to the template using a polymerase and a second mixture of nucleotide triphosphates, wherein one of the nucleotide triphosphates in the second mixture is blocked with a second blocking group and the remaining nucleotides are blocked with the first blocking group. When the rephasing cycles are done, the second blocking group is then unblocked to complete the process.

In the context of DFR, the terms "dinucleotide", "2mer", and "doublet" are used interchangeably. Any combination of two nucleotides can be used, including nucleotide repeats, for a total of 16 possible choices. A good choice is CA, wherein the nucleotide blocked with the first blocking group is C, and the nucleotide blocked with the second blocking group is A. Some other doublets may be less preferred because of a lower frequency of occurrence in the human genome. This means that more rephasing steps may be needed to achieve the same degree of concordance.

In the second mixture in the DFR process, where one nucleotide is blocked with the second blocking group, the other nucleotides may be blocked as well, using the first blocking group. In this case, the final step includes unblocking both the first and the second blocking groups. By way of example, the first blocking group may be an O-azidomethyl, and the second blocking group may comprise a ONH₂ group.

Another rephasing method is oligonucleotide rephasing ("Method four"). The method comprises performing multiple cycles of rephasing, wherein each cycle includes: extending the primer in a manner that is complementary to the template using a polymerase and a mixture of nucleotide triphosphates, wherein one of the nucleotide triphosphates is a nucleotide triphosphate that is blocked with a first blocking group, until substantially all of the copies are blocked; then unblocking the first blocking group; and extending the primer in a manner that is complementary to the template by ligating to the sequencing primer a 5' phosphorylated oligonucleotide that is blocked at the 3' end with a second blocking group. After the cycles are done, the rephasing is completed by unblocking the 5' phosphorylated oligonucleotide.

In one approach, the phosphorylated oligonucleotide has the formula U(N)_{Z}-X, where U is uracil, X is a blocking structure, Z is 6-20, and (N)z is a degenerate oligonucleotide and X is a non-reversible blocking structure. The non-reversible blocking structure may be inverted dT (IDT) incorporated at the 3'-end of the oligonucleotide, thereby creating a 3'-3' linkage which inhibits both degradation by 3' exonucleases and extension by DNA polymerases. The 5' phosphorylated oligonucleotide may be unblocked by treating with an enzyme mixture of Uracil-DNA Glycosylase (UDG) and apurinic/apyrimidinic endonuclease 1 (Ape1) or similar abasic site endonuclease to cleave and remove the uracil base.

Using any of these approaches, more cycles or rounds of rephasing increases the number of clonal populations in which growing strands are more than 90% phase or more than 99% in phase. Typically, a rephasing module includes 5 to 15 or 5 to 10 rephasing rounds or cycles, such as 5, 6 or 7 rounds.

Any of these rephasing methods can optionally be preceded by readjusting the site backwards up the chain being sequenced. This trims the most recent bases added to the primer, and provides a degree of sequence overlap. If done, the readjusting or cut-back is generally 5 to 50 bases, typically at least 10, 20, or 30 bases.

One way the readjusting can be done is by incorporating uracil residues during some of the sequencing steps preceding the rephasing, corresponding in length to the cutback window. The primer is extended in a manner that is complementary to the template using a polymerase and a mixture of nucleotide triphosphates, wherein one of the nucleotide triphosphates is a uracil. The primer is then cleaved at incorporated uracil bases: for example, using an enzyme mixture of Uracil-DNA Glycosylase (UDG) and apurinic/apyrimidinic endonuclease 1 (Ape1) or similar abasic site endonuclease.

Another method of readjusting the site of sequencing comprises extending the primer in a manner that is complementary to the template using a polymerase and a mixture of nucleotide triphosphates, wherein one of the nucleotide triphosphates is an RNA base or a thiolated nucleotide; then cleaving the primer at incorporated RNA bases. Another method of readjusting the site comprises treating the primer with a controlled 3' exonuclease, or with a nicking enzyme in a manner that is sequence dependent.

When the rephasing is completed, the copies of the target fragment in each amplicon will typically be at least 90% in phase, preferably 97%, 99%, or essentially 100% in phase. The number of rephased amplicons that are 100% in phase may be at least 70%, 80%, 90%, or 95% of the amplicons treated, as illustrated in the drawings.

Following the rephasing, the cycles of base-by-base sequencing can be resumed. Thus, the technology provided in this disclosure can be used to obtain long sequencing reads from a plurality of copies of a target nucleic acid, the method comprising performing multiple cycles of sequencing in which the sequencing primer in each copy is extended by one nucleotide, thereby identifying the complementary nucleotide in the template; after a number of such sequencing cycles, rephasing the nucleic acid copies; and then resuming cycles of the sequencing to identify further nucleotides in the template.

The rephasing can be done as many times as are necessary to achieve the accuracy and signal intensity desired, depending on the error rate of a particular sequencing methodology that takes copies out of phase to begin with. As exemplified below, rephasing is done at least once, and may be done 1-10 or 2-4 times per sequence read, which may comprise at least 200, 400, 800, or 1200 sequencing cycles.

The benefits of the rephasing include extension of the number of sequencing cycles having a discordance less than a certain percentage, as illustrated below in the working examples. By way of example, as shown in the drawings, the number of cycles having a discordance of less than a 2% threshold may increase by at least 25%, or by at least 1.5-fold or 2-fold, depending on the initial error rate and the number of rephasing cycles and events. The rephasing may extend the number of sequencing cycles having a discordance percentage of less than 2% by at least 100, 200, or 400 cycles, or more.

This disclosure also provides the reader with kits, reagent combinations, and intermediate mixtures including any of the reagents and mixtures described explicitly or inherent in the illustrations below, optionally accompanied by instructions for performing rephasing according to the technology of this disclosure.

Further embodiments of the invention are described and illustrated in the description that follows and in the accompanying drawings.

### BRIEF DESCRIPTION OF THE DRAWINGS

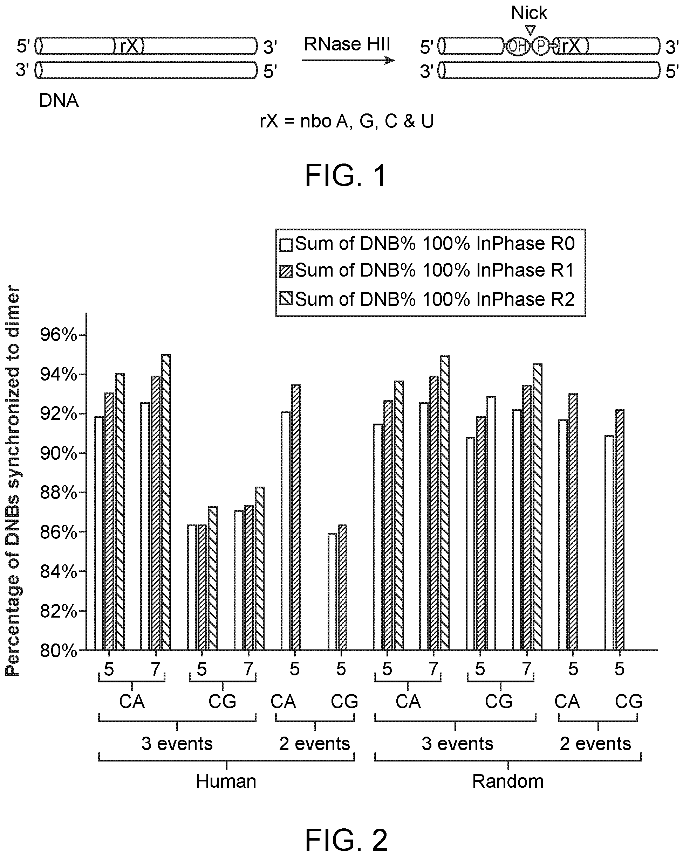

FIG. 1 depicts the use of RNAse HII to cleave the extending strand back to the first incorporated RNA base before rephasing. This is one of several "cutback" procedures explained below, in preparation for rephasing. RNAse HII catalyzes the cleavage of the DNA phosphodiester backbone 5' to ribonucleotide, or string of ribonucleotides, embedded within dsDNA leaving a 3' OH and 5' phosphate.
FIG. 2 shows the percentage of DNB templates that were 100% rephased back to the reference sequence using the dinucleotide approach, compared amongst different rephasing conditions. Each triplet shows the extent of rephasing after the first, second or third rephasing event. All conditions resulted in a rephasing of over 85% of the DNBs. Without any rephasing at all, clarify the number of DNBs in phase is virtually zero.

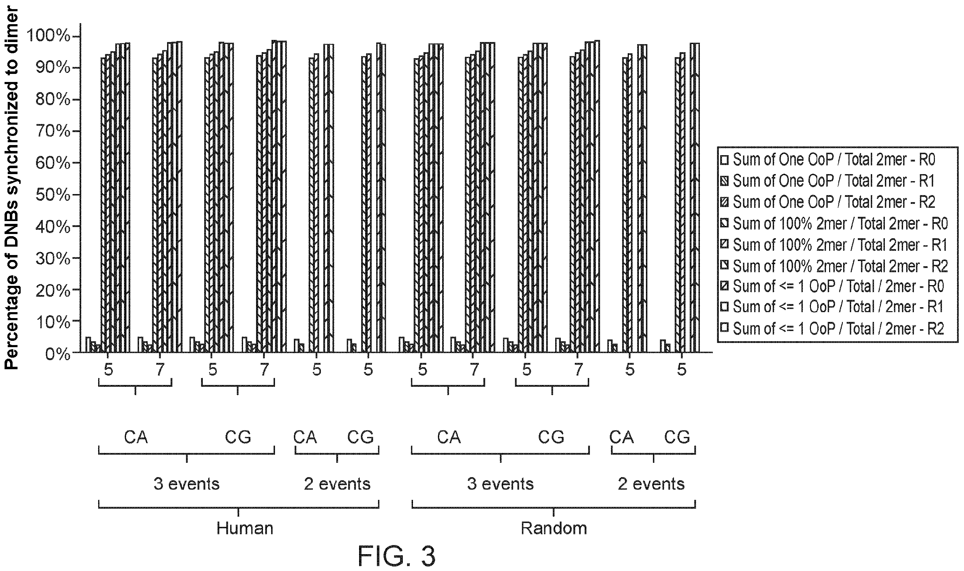

FIG. 3 shows the synchronized percentage of DNBs. The data illustrate how the percentage of DNBs that only have one site out of phase is the reverse of the percentage of DNBs that are fully in phase. While the percentage of fully synchronized DNBs increases between rephasing events, the percentage of DNBs with only one site out of phase decreases.

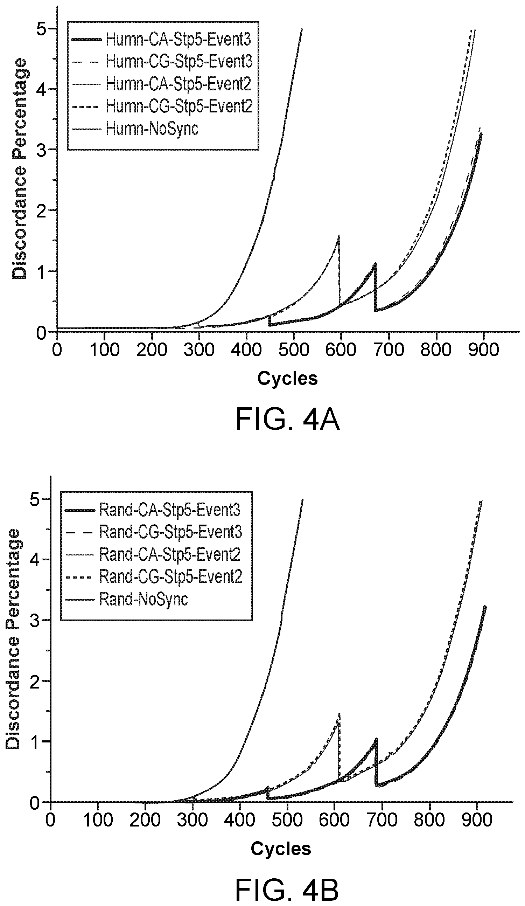

FIGS. 4A and 4B show the kinetics of phase discordance for a human reference sequence and a computer-generated random reference sequence, respectively. Under the conditions of the simulation, without rephasing, the discordance accumulates rapidly after the 300^{th} sequencing cycle, and is over 5% by the 500^{th} cycle. Two rephasing events keeps the discordance below 2% for over 750 cycles. Three rephasing events keeps the discordance below 2% for over 900 cycles

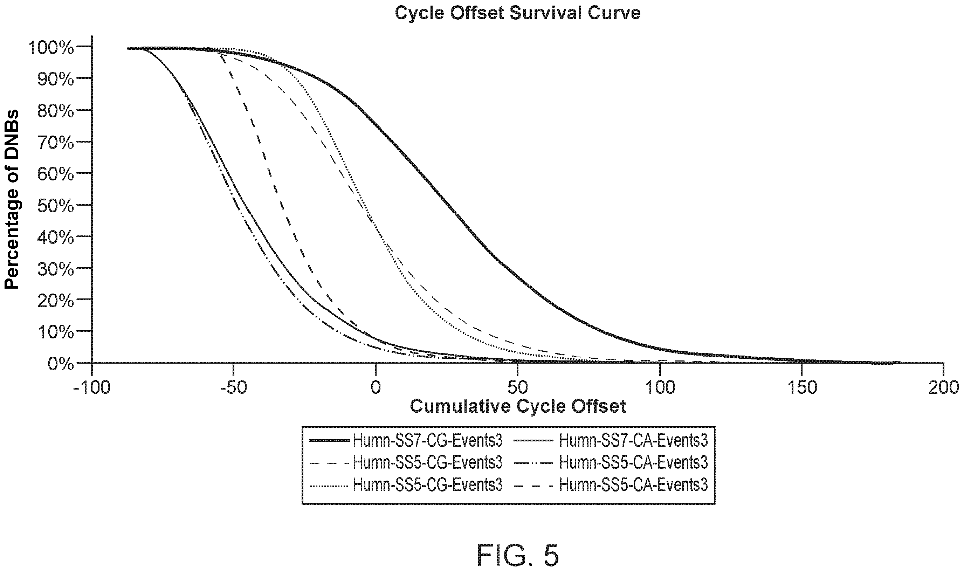

FIG. 5 shows the cumulative cycle offset after the final rephasing event. A negative value corresponds to the generation of overlapping sequence regions during rephasing, while a positive value corresponds to sequencing past the allotted number of cycles. The CG doublet results in 40% of the DNBs sequencing past the end point, compared with only about 8% for the CA doublet. The difference is attributed to the higher frequency of CA doublets in the human genome, compared with CG doublets.

### DETAILED DESCRIPTION

The methods disclosed herein can be employed for rephasing multiple copies of nucleic acids for any purpose. The technology is particularly applicable to nucleic acid sequencing methods such as array-based massively parallel sequencing using sequencing by synthesis.

Massively parallel sequencing-by-synthesis is generally carried out using DNA arrays in which cycles of template-directed DNA synthesis are carried on an array comprising numerous clonal populations of templates immobilized at physically separate positions on a substrate. Examples of clonal populations include, but are not limited to, (i) concatemers with many copies of a template sequence and (ii) clusters of many copies of a linear polynucleotide (for example, generated using bridge PCR). Sequencing using clonal populations (multiple copies of template) increases signal strength and mitigates against errors that may arise due to unexpected reactions occurring on individual copies.

### I. TERMS AND DEFINITIONS

In the description below, the following terms are used:

"Clonal population" refers to one or more template molecules, where the clonal population includes many copies (a population) of a sequence corresponding to the same (clonal) target sequence, such that the copies are physically or spatially associated with each other, e.g., contained or immobilized at a discrete position on a substrate, on separate beads, or in separate compartments (e.g., droplets). A template sequence and target sequence correspond as reverse complements of each other. Accordingly, the template sequence corresponding to a target sequence can be referred to as a "target sequence complement." In array based MPS, up to 10⁹ or more spatially separated clonal populations, each with hundreds or thousands of copies of template sequence, may be distributed on or positioned on a substrate. One example of a clonal population is a DNA nanoball (DNB) which is a single-stranded concatemer with many copies of a target sequence, typically produced by rolling circle amplification. R. Drmanac et al., Science. 327, 78-81, 2010. Another example of a clonal population is an amplicon cluster containing hundreds to thousands of amplicons with the same target sequence, typically produced by bridge amplification (e.g., PCR). In another example, clonal populations are attached to the surfaces of a beads (produced, for example by emulsion PCR; see (Metzker et al., Nat Rev Genet. 11 (1): 31-46, 2010). In some methods a clonal population may contain many copies of a target sequence and its complement. Clonal populations are generally prepared from a "library," such as a genomic or cDNA library.

"Template," "template sequence," "nucleic acid template" and the like refer to a polynucleotide recognized by a nucleic acid polymerase (e.g., DNA polymerase). In MPS a library of templates are prepared from fragments of DNA of molecules of interest (e.g., genomic DNA) linked to adaptor sequences. As known in the art and discussed elsewhere herein, the polymerase catalyzes formation of a complementary polynucleotide strand (a "growing strand," "extended duplex," "extending strand," or "extended primer") by extending a primer hybridized to the template (typically to an adaptor sequence) by successive addition of (deoxy)ribonucleotides, where each added nucleotide forms a base pair with (i.e., is complementary to) the corresponding base of the template.

"Target sequence" refers to a nucleic acid sequence (generally a DNA sequence) that is determined in a sequencing reaction. The target sequence (sometimes called a "Reference Sequence") is complementary to, and produced by replication of, a corresponding DNA template.

"Nucleobase" means a nitrogenous base that can base-pair with a complementary nitrogenous base of a template nucleic acid. Exemplary nucleobases include adenine, cytosine, guanine, thymine, uracil, inosine and derivatives of these. References to thymine refer equally to uracil unless otherwise clear from context. The terms "nucleobase," "nitrogenous base," add "base" are used interchangeably.

A "nucleotide" consists of a nucleobase, a sugar, and one or more phosphate groups. They are monomeric units of a nucleic acid sequence. In RNA, the sugar is a ribose, and in DNA a deoxyribose. The nitrogenous base is a derivative of purine or pyrimidine. The purines are adenine (A) and guanine (G), and the pyrimidines are cytosine (C) and thymine (T) (or in the context of RNA, uracil (U)). The C-1 atom of deoxyribose is bonded to N-1 of a pyrimidine or N-9 of a purine. A nucleotide is also a phosphate ester or a nucleoside, with esterification occurring on the hydroxyl group attached to C-5 of the sugar. Nucleotides are usually mono, di- or triphosphates. A "nucleoside" is structurally similar to a nucleotide, but does not include the phosphate moieties. Common abbreviations include "dNTP" for deoxynucleotide triphosphate.

"Nucleic acid" means a polymer of nucleotide monomers. The terms may refer to single- or double-stranded forms. Monomers making up nucleic acids and oligonucleotides are capable of specifically binding to a natural polynucleotide by way of a regular pattern of monomer-to-monomer interactions, such as Watson-Crick type of base pairing, base stacking, Hoogsteen or reverse Hoogsteen types of base pairing to form duplex or triplex forms. Such monomers and their internucleosidic linkages may be naturally occurring or may be analogs thereof. Non-naturally occurring analogs may include peptide nucleic acids, locked nucleic acids, phosphorothioate internucleosidic linkages, bases containing linking groups permitting the attachment of labels, such as fluorophores, or haptens. Nucleic acids typically range in size from a few monomeric units, when they are usually referred to as "oligonucleotides," to several hundred thousand or more monomeric units.

Whenever a nucleic acid (other than a template) or oligonucleotide is represented by a sequence of letters (upper or lower case), such as "ATGCCTG," the nucleotides are in 5' to 3' order from left to right and that "A" denotes deoxyadenosine, "C" denotes deoxycytidine, "G" denotes deoxyguanosine, and "T" denotes thymidine, "I" denotes deoxyinosine, "U" denotes uridine, unless otherwise indicated or obvious from context. As will be understood by the skilled reader, where a template sequence is shown aligned to a target the template is the reverse complement of the target and is represented in the 3'→5' orientation. See "Scheme A" below. Unless otherwise noted, the terminology and atom numbering conventions will follow those disclosed in Strachan and Read, Human Molecular Genetics 2 (Wiley-Liss, New York, 1999). Usually nucleic acids comprise the natural nucleosides (deoxyadenosine, deoxycytidine, deoxyguanosine, deoxythymidine for DNA or their ribose counterparts for RNA) linked by phosphodiester linkages; however, they may also comprise non-natural nucleotide analogs, such as modified bases, sugars, or internucleosidic linkages. Selection of appropriate composition for the oligonucleotide or nucleic acid substrates may be guided by treatises, such as Sambrook et al., Molecular Cloning, Second Edition (Cold Spring Harbor Laboratory, New York, 1989).

"Polynucleotide" is used interchangeably with the term "nucleic acid" to mean DNA, RNA, and hybrid and synthetic nucleic acids and may be single-stranded or double-stranded. "Oligonucleotides" are short polynucleotides of between about 6 and about 300 nucleotides in length. "Complementary polynucleotide" refers to a polynucleotide complementary to one strand of a nucleic acid.

A "reversible terminator" nucleotide is a nucleotide analog that can be incorporated into a growing strand by a polymerase and which comprises a blocking group (sometimes at the 3'-OH position of deoxyribose). The blocking group prevents formation of a phosphodiester bond between the nucleotide at the 3' terminus of a growing strand and an unincorporated nucleotide, and reversibly terminates further extension of the growing strand. In some cases, a reversible blocking group is a chemical moiety attached to the 3'-O position of the nucleotide sugar moiety. A reversible blocking group can be cleaved by an enzyme (such as a phosphatase or esterase), a chemical reaction, exposure to heat, light, etc., to provide a hydroxyl group at the 3' position of the nucleoside or nucleotide such that addition of a nucleotide by a polymerase may occur. The blocking group prevents polymerization and addition of nucleotides to the 3' terminus of the growing strand. Removal of the blocking group allows polymerization to continue. A reversible terminator nucleotide can be referred to as "blocked nucleotide," which may be "unblocked" by removal of the blocking group. The terms "reversible blocking group," "removable blocking group," "blocking moiety," a "blocking group," "reversible terminator blocking group" and the like are used interchangeably. Unless otherwise apparent from context, the terms reversible terminator nucleotide," "reversible terminator," "RT," and "nonlabeled reversible terminator (NLRT)," refer to a sequencing reagent comprising a nucleobase or analog, deoxyribose or analog, phosphate, and a cleavable (or otherwise removable) blocking group. Reversible terminators may be labeled (e.g., to a fluorescent dye via a cleavable linker) or unlabeled (NLRT; see US Pat. No. 10,851,410). The terms "reversible," "removable," and "cleavable" in reference to a blocking group are used interchangeably. In some cases a growing strand or an oligonucleotide in which the 3'-prime terminal nucleotide is blocked can be referred to as "blocked," as will be clear from context.

As used herein "unblocked nucleotides" refers to nucleotides that can be incorporated into a growing strand. Unblocked nucleotides may be the "natural" or naturally occurring nucleotide monophosphates (N), such as deoxyadenosine monophosphate (A), thymidine monophosphate (T), deoxyguanosine monophosphate (G), deoxycytidine monophosphate (C), deoxyuridine monophosphate (U), or their cognate triphosphate forms (dATP, dTTP, dUTP, dCTP, dGTP), unblocked analogs, and the like. If not otherwise specified or clear from context, reference to a "nucleotide" can mean a naturally occurring nucleotide, a nucleotide analog used in sequencing, a blocked nucleotide or an unblocked nucleotide.

As described herein, a degenerate oligonucleotide used in rephasing can be used to block extension of a growing strand, and removal of the oligonucleotide is a type of "unblocking."

The term "subunit" is sometimes used to refer to the growing strand (or corresponding portion of a template, such as monomers in a concatemer) that comprises one copy of a target sequence) as will be apparent from context, and may include associated adaptors, primer binding sequences and the like.

"Substantially all" in reference to the proportion of extended primers or growing strands in a clonal population that are blocked or that are blocked at the same reference position means that more than 90%, preferably more than 95%, 96%, 97%, 98%, 99%, or 99.5% (and sometimes 100%) are blocked. The proportion of blocked strands can be determined emperically or can be estimated mathematically based on knowledge of a template sequence, the number of rephasing modules and the like. As used herein, "most" and "a majority" means 51% or more.

A "primer" is an oligonucleotide that is capable, upon forming a duplex with a polynucleotide template, of acting as a point of initiation of nucleic acid synthesis and being extended from its 3' end along the template so that an extended duplex is formed. It will be recognized that a complex including an extended primer, or growing strand, annealed to a template sequence can be referred to as a duplex. The terms "probe," "primer" and "oligonucleotide primer," are used interchangeably in this disclosure to refer to oligonucleotides that anneal to a complementary sequence of a nucleic acid template and can be extended by a polymerase by addition of nucleotides. A primer to which nucleotide(s) have been added is a "growing strand," 'extended duplex," or "extended primer." When a dNTP (i.e., nucleoside triphosphate) is added to the 3' terminus of the primer, pyrophosphate is removed such that a nucleoside monophosphate (or nucleotide) is incorporated. An unlabeled or no labeled reversible terminator nucleotide can refer to either form (free nucleoside triphosphate or incorporated nucleotide monophosphate), unless otherwise specified, as will be clear from context. An unlabeled, or no labeled reversible terminator, nucleotide can be referred to as an NLRT. The sequence of nucleotides added during the extension process are determined by the sequence of the template polynucleotide. Usually primers are extended by a DNA polymerase. Primers usually have a length in the range of from 9 to 40 nucleotides, or from 14 to 36 nucleotides.

In the context of dinucleotide frequency rephasing (DFR), the terms dinucleotide, "2mer", and "doublet" are used interchangeably. Unless otherwise specified or required, the DFR method can be used using any combination of two nucleotides, including nucleotide repeats, for a total of 16 possible choices.

The term "sequentially sequenced" is used in this disclosure to refer to any method of nucleic acid sequencing that comprises a sequence of cycles in which one or more bases at one position in the template nucleic acid are determined, and the method then passes to the base or bases that are adjacent until all the bases in a particular region of the template have been determined. Sequencing by synthesis is exemplary, in which determination of single bases is determined by synthesizing a complementary strand in sequential cycles base by base, determining the base added in each cycle.

"Amplicon" means the product of a polynucleotide amplification reaction, namely, a population of polynucleotides that are replicated from one or more starting sequences. Amplicons may be produced by a variety of amplification reactions, including but not limited to polymerase chain reactions (PCRs), bridge PCR, linear polymerase reactions, nucleic acid sequence-based amplification, rolling circle amplification (U.S. Pat. Nos. 7,115,400, 4,683,195; 5,210,015; 6,174,670; 5,399,491; 6,287,824 and 5,854,033; and U.S. Pub. No. 2006/0024711).

"Array" or "microarray" means a solid support (or collection of solid supports such as beads) having a surface, preferably but not exclusively a planar or substantially planar surface, which carries a collection of sites comprising nucleic acids such that each site of the collection is spatially defined and not overlapping with other sites of the array; that is, the sites are spatially discrete. The array or microarray can also comprise a non-planar interrogable structure with a surface such as a bead or a well. The oligonucleotides or polynucleotides of the array may be covalently bound to the solid support, or it may be non-covalently bound. Conventional microarray technology is reviewed in Schena, Ed. (2000), Microarrays: A Practical Approach (IRL Press, Oxford). As used in this disclosure, "random array" or "random microarray" refers to a microarray where the identity of the oligonucleotides or polynucleotides is not discernable, at least initially, from their location but may be determined by a particular biochemistry detection technique on the array. See U.S. Pat. Nos. 6,396,995; 6,544,732; 6,401,267; and 7,070,927; PCT publications WO 2006/073504 and 2005/082098; and U.S. Pat. Pub. Nos. 2007/0207482 and 2007/0087362.

"Solid support" and "support" refer to a material or group of materials having a rigid or semi-rigid surface or surfaces. Microarrays usually comprise at least one planar solid phase support, such as a glass microscope slide.

"Incorporate" means becoming part of a nucleic acid molecule. In SBS, incorporation of an RT occurs when a polymerase adds an RT to a growing DNA strand through the formation of a phosphodiester or modified phosphodiester bond between the 3' position of the pentose of one nucleotide, that is, the 3' nucleotide on the DNA strand, and the 5' position of the pentose on an adjacent nucleotide, that is, the RT being added to the DNA strand.

"Label," in the context of a labeled affinity reagent, means any atom or molecule that can be used to provide a detectable and/or quantifiable signal. Suitable labels include radioisotopes, fluorophores, chromophores, mass labels, electron dense particles, magnetic particles, spin labels, molecules that emit chemiluminescence, electrochemically active molecules, enzymes, cofactors, and enzyme substrates.

"Restoring phase," "resetting phase," and "rephasing" are used interchangeably in this disclosure.

"Repositioning" or "cutback" is a process used in conjunction with rephasing to back the position of sequencing 5 to 50 bases upstream in the growing strand. As described below, this provides the user with overlap of sequencing information obtained before and after a rephasing event, rather than creating a gap.

As used in this disclosure and in the appended claims, the singular forms "a," "an," and "the" include plural referents unless the context clearly dictates otherwise. Thus, for example, reference to "a polymerase" refers to one agent or mixtures of such agents, and reference to "the method" includes reference to equivalent steps and/or methods.

Unless otherwise stated or required, the other technical and scientific terms used in this disclosure have their ordinary meaning.

Where a range of values is provided, each intervening value, to the tenth of the unit of the lower limit unless the context clearly dictates otherwise, between the upper and lower limit of that range and any other stated or intervening value in that stated range is encompassed. The upper and lower limits of these smaller ranges may independently be included in the smaller ranges is also encompassed, subject to any specifically excluded limit in the stated range. Where the stated range includes one or both of the limits, ranges excluding either both of those included limits are also included.

The practice of the technology put forth in this disclosure may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example. Conventional techniques and descriptions can be found in standard laboratory manuals such as Genome Analysis: A Laboratory Manual Series (Vols. I-IV), Using Antibodies: A Laboratory Manual, Cells: A Laboratory Manual, PCR Primer: A Laboratory Manual, and Molecular Cloning: A Laboratory Manual (all from Cold Spring Harbor Laboratory Press), Stryer, L. (1995) Biochemistry (4th Ed.) Freeman, N.Y., Gait, "Oligonucleotide Synthesis: A Practical Approach" 1984, IRL Press, London, Nelson and Cox (2000), Lehninger, Principles of Biochemistry 3rd Ed., W. H. Freeman Pub., New York, N.Y. and Berg et al. (2002) Biochemistry, 5th Ed., W. H. Freeman Pub., New York, N.Y.

### II. SEQUENCING-BY-SYNTHESIS USING REVERSIBLE TERMINATORS

The technology of this invention is generally applicable to sequencing methods such as sequencing-by-synthesis (SBS) using reversible terminators. In these methods one nucleotide (typically) is identified per sequencing cycle. Various SBS methods are known. See, for example, R. Drmanac et al., Science. 327, 78-81, 2010; PCT Pat. Pub. WO 2016/133764; Mardis E., 2017, Nature Protocols Nature Protocols 12:213-218; Margulies et al., 2005, Nature 437:376-380; Ronaghi et al., 1996, Anal. Biochem. 242:84-89; Constans, A, 2003, The Scientist 17(13):36; and Bentley et al., 2008, Nature 456(7218):53-59. Determining the sequence of a nucleic acid typically entails performing multiple cycles of a reaction that generates a signal that corresponds to the identity of one or more nucleotides in the sequence. In one approach this is accomplished using primer extension reactions on a clonal population comprising many copies of the sequence to be determined. In some approaches, primer extension incorporated dNTP analog(s) (reversible terminators) are labeled with a fluorescent dye. In some approaches, the dNTP analog(s) that is incorporated is not linked to a dye is detected by affinity reagents (e.g., antibody sequencing). See U.S. Patent No. 10,851,410, describing CoolMPS^{®}. Sequencing arrays comprising a large number (often hundreds of millions) of positions are routinely used and are generally contained in a flow cell designed for used in automated sequencing devices. Arrays may be patterned or sites of template attachment may be randomly positions on a substrate. DNA sequencers that perform sequencing by synthesis are commercially available, for example, the BGISEQ-500 from BGI, (Shenzhen, PRC) and NextSeq from Illumina Inc. (San Diego, CA). Some SBS methods include detection of a proton released upon incorporation of a nucleotide into an extension product. For example, sequencing based on detection of released protons can use an electrical detector and associated techniques that are commercially available from Ion Torrent (Guilford, Conn., a Life Technologies subsidiary) or sequencing methods and systems described in U.S. Pat. App. Pub. Nos. 2009/0026082 A1; 2009/0127589 A1; 2010/0137143 A1; or 2010/0282617 A1.

### III. PHASE

In a sequencing reaction applied to a clonal population, growing strands are extended in each cycle, typically by addition of one nucleotide per sequencing cycle. Two growing strands in a clonal population are "in phase" at any sequencing cycle when the strands comprise the same number of incorporated nucleotides (e.g., corresponding to the number of incorporation cycles of the sequencing reaction) and terminate at the same position of the corresponding template sequence. Growing strands that are "in phase" will have the same base seqence at the 3-prime terminus.

### IV. LOSS OF PHASE/ DISCORDANCE

Sequencing of a clonal population provides advantages such as a stronger signal, improves accuracy, and, in some methods, reduces amplification errors that occur in the course of producing the clonal population. However, such sequencing requires the coordinated extension of the templates in each clonal population. That is, all or most of the growing strands in a clonal population must be extended in each cycle, and by the same number of nucleotides (typically one nucleotide) per cycle. However, in practice incorporation of nucleotides into numerous growing strands at any given position on an array can "fall out of phase." This refers to the fact that, in any given sequencing cycle in which nucleotides are incorporated into growing strands in a clonal population, there may be non-uniformity in the sequencing chemistry resulting in some growing strands in which no nucleotide is incorporated and/or some growing strands in which more than one nucleotide is incorporated. For example, there is a small frequency of sequence reactions for each consecutive nucleotide that do not feed into the next sequencing cycle in a normal manner. A sequencing cycle that does not go to completion may yield back the same intermediate produced from the previous cycle. In this case, the next cycle will identify the nucleotide that should have been identified a cycle earlier. That particular copy of the DNA will be one cycling phase behind the other copies (lag sequencing). Alternatively, a sequencing cycle that skips a nucleotide or processes two nucleotides in the same cycle will be one cycle ahead of the other copies (run-on sequencing). A loss (or decrease) in phase can be referred to as an increase in discordance.

The term "discordance" is usually used in the sequence arts to indicate that a base, as called by the sequencing, as not matching the known reference identity of the base at that position in the read. As used herein, discordance refers to the proportion of strands in a clonal population that are out of phase. Percent discordance refers to the percentage of calls at a position that do not match the reference sequence, and is an indicator of the proportion of growing strands that are out of phase (there is increased probability of making a discordant call as more primers become out of phase). Calls may not be discordant if only some growing strands are out of phase if the mixing of intensities does not reach the state of "flipping" the position into the wrong call. However, even if not reflected in a miscalled read reducing the number of out-of-phase strands increases signal strength, allows longer reads and has other benefits.

The consequence of individual copies of an amplified DNA (e.g., a target sequence) going out of phase is that the information obtained from that copy of the DNA or target sequence will be incorrect in subsequent cycles of sequencing reaction. A cyclical sequencing process will thus accumulate more and more out of phase copies as sequencing continues, with the proportion of copies correctly in phase decreasing over time.

Depending on the sequencing methodology and reagents being used, we have found that after 200 cycles typically about 20% of template copies may lag behind, and about 10% are ahead of phase. This will have the effect of decreasing signal-to-noise ratio, blurring the readout, until the information is no longer sufficiently accurate. This effectively limits the length of the sequence read that can be obtained. The technology of this invention provides the user with an opportunity to rephase the DNA amplicons between sequencing cycles.

"Schemes," such as "Scheme A" below, are used in this disclosure to describe clonal populations in which individual growing strands (s1-4) are, or are not, in phase. The "target sequence" is sometimes called the "reference sequence." The target sequence is complementary to template sequence. Importantly, the template and target sequences do not necessarily (and generally do not) represent the entirety of the template or target nucleotides in a clone or in a growing strand (extended primer). For example, in Scheme A below, the 5'-most C of the sequence shown (cgta...) may be the 50^{th}, 100^{th}, 200^{th}, etc. nucleotide in an extended primer (wherein the first incorporated nucleotide extending the primer is deemed nucleotide 1). For clarity and convenience we adopt the convention of referring to the the 5'-most nucleotide (C) as a nucleotide incorporated in the *first* sequencing cycle, rather than the 200^{th}, for example).

### SCHEME A

| | |
|---|---|
| Template | gcatgcatgcatgcatgcatgcatgcaatgct (SEQ ID NO: 1) |
| Target | cgtacgtacgtacgtacgtacgtacgttacgt (SEQ ID NO: 2) |
| s1 | cgtacgtacgtacgta (SEQ ID NO: 3) |
| s2 | cgtacgtacgtacgt (SEQ ID NO: 4) |
| s3 | cgtacgtacgtacgt (SEQ ID NO: 4) |
| s4 | cgtacgtacgtacg (SEQ ID NO: 5) |

In Scheme A, Strands s2 and s3 show the sequence of in-phase growing strands after 15 sequencing cycles, for which the nucleotide incorporated in the next cycle will be A. s1 shows the sequence of a growing strand in which an extra nucleotide is incorporated, and for which the nucleotide incorporated in the 16^{th} cycle will be C (i.e., run-on sequencing). s4 shows the sequence of growing strands in which no nucleotide is incorporated in the 15^{th} cycle, and for which the nucleotide incorporated in the 16^{th} cycle will be T (i.e., lag sequencing).

Scheme A The following example shows the correct or reference sequence of the 3' region of a fully extended growing strand, and four examples showing the sequences of the last 15 rounds of extension (15 sequencing cycles) of growing strands for four of the subunits. Individual growing strands are sometimes called "subunits." It will be understood that the illustration below involves one clonal population (e.g., one 'spot' on an array) and that the rephasing steps described here occur on an array of thousands, millions or billions of clonal populations with different sequences.

In this illustration some subunits may terminate at a T. s2 and s3 reflect accurate incorporation of 15 nucleotides in 15 sequencing cycles. In this example one subunit, s4, is lagging by one base having incorporated a G at this cycle. Subunit s1 has run-on relative to s2 and s3 and terminates with an A.

### V. REPHASING; RESTORATION OF PHASE

This disclosure provides methods and reagents for restoring phase in stepwise sequencing of clonal DNA using reversible chain terminators (RTs). Removing lag (typically -1 or -2 bases) and run-on (usually +1 base) sequencing copies that have accumulated (particularly after one hundred or more cycles of sequencing) is needed for high quality long sequence reads longer than 300 or 500 bases. Rephasing the growing strands at sites on the array will improve sequence quality and read length. Rephasing at 20% or more, 30%, or more than 50% or more than 60%, or preferably more than 70%, or 80% of DNA clones being sequenced on an array would provide sufficient yield for long MPS reads (500 bases to 700 bases or 600 bases to 1000 bases or even longer). In this context, the percentage rephasing refers to the percentage of clonal structures in which the individual extended strands of the subunits of the structure have become reset to the same sequence length and have terminated at the same position in the target fragment representing the clonal structure such as a DNB.

### VI. METHOD 1: SINGLE-NUCLEOTIDE FREQUENCY RE-PHRASING WITH RUN-FORWARD USING BLOCK-1 (Example not encompassed by the wording of the claims)

This rephasing Method 1 comprises the incorporation of 3 natural nucleotides (for example A, C, G) plus one 3' reversibly blocked nucleotide (for example T). Scheme A shows an out-of-phase population (as described above) at completion of a standard sequencing cycle with cleavage of the terminal blocking group. Subunit s1 has run-on relative to s2 and s3 and terminates with an A. In this context, "subunits" can mean, without limitation, monomers in a concatemer or individual template polynucleotides in a cluster. Some subunits may terminate at a T and in this example one subunit is lagging by one base having incorporated a G at this cycle.

### SCHEME A

### Clonal Population 1

| | |
|---|---|
| Template | gcatgcatgcatgcatgcatgcatgcaatgct (SEQ ID NO: 1) |
| Target | cgtacgtacgtacgtacgtacgtacgttacgt (SEQ ID NO: 2) |
| s1 | cgtacgtacgtacgta (SEQ ID NO: 3) |
| s2 | cgtacgtacgtacgt (SEQ ID NO: 4) |
| s3 | cgtacgtacgtacgt (SEQ ID NO: 4) |
| s4 | cgtacgtacgtacg (SEQ ID NO: 5) |

In this method, the extended strand is allowed to run-forward with incorporation of 3 natural (i.e., unblocked), and 1 blocked nucleotide mix. Each of the subunits will terminate at a T in this example. However, some of the subunits will terminate at Ts that are at different positions in the sequence, thus not achieving the desired effect of all subunits being in phase. This is because a T was the terminating base in some of the subunits.

To "restore phase," the growing strands are allowed to run-forward with incorporation of 3 natural and 1 blocked nucleotide mix for example using polymerase and a nucleotide mixture with 1 blocked nucleotide and 3 unblocked (e.g., natural) nucleotides. The blocked nucleotide can be any of the 4 nucleotides: In this example T is blocked (denoted "T*"). Extension or "run-on" of the Scheme A population results in the Scheme B population shown below. Incorporated bases are shown in underlined, upper case bold font.

### SCHEME B

### Clonal Population 1 Following Run-On with Blocked T (T*) and 3 natural nucleotides

| | |
|---|---|
| Template | gcatgcatgcatgcatgcatgcatgcaatgct (SEQ ID NO: 1) |
| Target | cgtacgtacgtacgtacgtacgtacgttacgt (SEQ ID NO: 2) |
| s1 | cgtacgtacgtacgt**ACGT*** (SEQ ID NO: 6) |
| s2 | cgtacgtacgtacgt**ACGT*** (SEQ ID NO: 6) |
| s3 | cgtacgtacgtacgt**ACGT*** (SEQ ID NO: 6) |
| s4 | cgtacgtacgtacg**T*** (SEQ ID NO: 7) |

In this example, only T is blocked. Growing strands in each clonal population on an array will be extended until an A appears in the template, at which point incorporation of T will terminate extension of the individual strand (i.e., all subunits have terminated at a T since the T was the only nucleotide to have a 3' blocking group). While in Scheme A two of four of the subunits (s1 and s4) are out of phase, following rephasing only one of the four is out of phase in Scheme B, a reduction in discordance. Subunit S1 is now in phase relative to S2 and S3. Subunit s4 is now further out of phase because the original phasing straddled a "T" that was the terminating nucleotide.

It will be recognized that, in this example, no sequence information is available for positions 16, 17 and 18 (ACG). This gap in information may be addressed in various ways, as discussed below.

Scheme C illustrates effect of rephasing of the same Scheme A clonal population (i.e., the same target sequence), but using C as the blocked nucleotide.

### SCHEME C

### Clonal Population 1 Following Run-On with Blocked C (C*) and 3 natural nucleotides

| | |
|---|---|
| Template | gcatgcatgcatgcatgcatgcatgcaatgct (SEQ ID NO: 1) |
| Target | cgtacgtacgtacgtacgtacgtacgttacgt (SEQ ID NO: 2) |
| s1 | cgtacgtacgtacgta**C*** (SEQ ID NO: 8) |
| s2 | cgtacgtacgtacgt**AC*** (SEQ ID NO: 8) |
| s3 | cgtacgtacgtacgt**AC*** (SEQ ID NO: 8) |
| s4 | cgtacgtacgtaccg**TAC*** (SEQ ID NO: 8) |

In this example, only C is blocked and the growing strands in each clonal population will be extended until a G appears in the template, at which point incorporation of C* will terminate extension of the individual strand. While in Scheme A two of four of the subunits (s1 and s4) are out of phase, in Scheme C all of s1-4 is in phase, a reduction in discordance.

Scheme D illustrates extension of a different clonal population, "Clonal Population 2," (having a different target sequence than the target sequence of Scheme A). As for Scheme C, T is used as the blocked nucleotide in the rephasing step. In Scheme D, two of the four strands is in phase.

### SCHEME D

### Clonal Population 2 Following -On With Blocked T

| | |
|---|---|
| Template | gcatgcatgcatgcaatttatttatttag (SEQ ID NO: 9) |
| Target | cgtacgtacgtacgttaaataaataaatc (SEQ ID NO: 10) |
| s1 | cgtacgtacgtacgtt**AAAT*** (SEQ ID NO: 11) |
| s2 | cgtacgtacgtacgt**T*** (SEQ ID NO: 12) |
| s3 | cgtacgtacgtacgt**T*** (SEQ ID NO: 12) |
| s4 | cgtacgtacgtac**GT*** (SEQ ID NO: 7) |

In this rephasing method, in each rephasing event one nucleotide is blocked and three are not blocked. Although any one of the 4 nucleotides may be selected as blocked, the efficiency of the method may vary based on the nucleotide composition of the target sequences (e.g., such as the differences between a mammalian genomic sequence and a bacterial genomic sequence, of a high GC library compared to a low GC library, for example). The rephasing result for any given clonal population is dependent on the template sequence and the blocked nucleotide selected in a "restoration" cycle. However, in an array with a large number of populations these is generally a net gain in phase. In general, the ability of Method 1 to rephase a strand correctly is limited by the frequency of the blocked nucleotide (T or C in the examples above) in the target sequence.

To increase the proportion of out-of-phase strands returned to phase, or to achieve a higher probability of restoring all subunits into phase, the frequency of the terminating feature in the target sequence or template should be less common. This is accomplished by stopping the run-forward steps not at a single nucleotide in the template, but at a selected dinucleotide. Methods 2 - 4, described below, use this approach.

### VII. METHOD 2: DINUCLEOTIDE-FREQUENCY REPHASING (DFR) WITH RUN-FORWARD

In this method, each growing strand is allowed to be extended until a position in the target sequence corresponding to a selected dinucleotide sequence. The dinucleotide sequence can also be referred to as a "doublet" or "2mer." Any dinucleotide selected from the 16 possible combinations may be used. For purposes of the following illustration the dinucleotide TC is recognized. In this illustration, the clonal population is represented as Scheme E, shown below.

### SCHEME E

| | |
|---|---|
| Target | cgtacgtacgtacgtacgtacgtacgtcacgtacgtacgt (SEQ ID NO: 13) |
| s1 | cgtacgtacgtacgta (SEQ ID NO: 3) |
| s2 | cgtacgtacgtacgt (SEQ ID NO: 4) |
| s3 | cgtacgtacgtacgt (SEQ ID NO: 4) |
| s4 | cgtacgtacgtacg (SEQ ID NO: 5) |

In one embodiment the steps of a DFR cycle are as described below. The ordinarily skilled practitioner will recognize that steps described herein are illustrative, but that specific reagents or methods may be varied when carrying out the method.

Step 1. Extend growing strands of clonal populations by incorporating four nucleotides, one of which (in this example, T) is blocked, and three of which (in this example, A, C, and G) are not blocked. The nucleotide that is blocked corresponds to the first nucleotide of the selected doublet. The blocked first nucleotide may be blocked with a group called "blocking group-1" which may be denoted as an asterisk (e.g., T*).

### SCHEME F

### Clonal Population 1 Dinucleotide (TC) Method Blocked T

| | |
|---|---|
| Target | cgtacgtacgtacgtacgtacgtacgtcacgtacgtacgt (SEQ ID NO: 13) |
| s1 | cgtacgtacgtacgta**CGT*** (SEQ ID NO: 6) |
| s2 | cgtacgtacgtacgt**ACGT*** (SEQ ID NO: 6) |
| s3 | cgtacgtacgtacgt**ACGT*** (SEQ ID NO: 6) |
| s4 | cgtacgtacgtacg**T*** (SEQ ID NO: 7) |

Step 2. Remove polymerase and excess (unincorporated) nucleotides. It will be recognized that removal can be accomplished using any suitable method and can generally be referred to as "wash to remove excess nucleotides and polymerase." In one approach "washing" is accomplished by flowing a buffer through a flow cell containing the array.

Step 3. Unblock the terminal group-1 blocking group with an unblocking agent to return the 3' group to a hydroxyl moiety able to accept further nucleotide extension. Thus, each of the subunits terminates at a T base, as illustrated in Scheme G. Methods for removing blocking groups or otherwise unblocking a reversible terminator are known in the art and discussed herein below.

### SCHEME G

| | |
|---|---|
| Target | cgtacgtacgtacgtacgtacgtacgtcacgtacgtacgt (SEQ ID NO: 13) |
| s1 | cgtacgtacgtacgtacgt (SEQ ID NO: 14) |
| s2 | cgtacgtacgtacgtacgt (SEQ ID NO: 14) |
| s3 | cgtacgtacgtacgtacgt (SEQ ID NO: 14) |
| s4 | cgtacgtacgtacgt (SEQ ID NO: 4) |

Step 4. Carry out an incorporation step by adding a reversibly terminated nucleotide with blocking group-2. The blocked nucleotide corresponds to the second base of the selected dinucleotide (C in this example). This step "reads" the next consecutive base, to see if it corresponds to (or correctly identifies) the second nucleotide (C) in the selected dinucleotide (TC). The blocked nucleotide corresponding to the second nucleotide of the dinucleotide has a blocking group ("blocking group 2") that is different from blocking group-1. Blocking group-2 may be denoted by a reverse arrow (e.g., C ◄ ). In addition to incorporating a nucleotide corresponding to the second base of the dinucleotide (C in this example) blocked with Blocking group 2, nucleotides corresponding to other three nucleotides (e.g. A, G, T), blocked with blocking group-1, are included to advance all strands by one base. By adding all 4 nucleotides there is advantageously less possibility of the C mismatching when the template is not G.

According to this method, blocking group-2 is not unblocked under the same conditions that unblock blocking group-1. For example, in some cases different reagents are used to remove the group-1 and group-2 blocking group, and block-2 cannot be un-blocked with the same reagent that can unblock group-1. Put differently, conditions may be selected under which blocking group-1 is removed and blocking-group-2 is not removed. For example, in one approach Block 1 is -O-azidomethyl which may be removed by phosphine (e.g., TCEP) cleavage and Block 2 is -O-NH₂, which may be removed by sodium nitrite cleavage. See Hutter et al., 2010, Nucleosides Nucleotides Nucleic Acids. 29(11) doi:10.1080/15257770.2010.536191. Many other groups are available including those comprising a disulfide bond. See discussion below. The method can be carried out using reversible terminators and conditions under which blocking group 1 is partially or fully un-blocked with the reagent that unblocks group-2. It will be recognized that multiple different blocking groups can be used (e.g., two different blocking groups can be substituted for blocking group 1 and/or two different blocking groups can be substituted for blocking group 2) provided that the relationship vis-à-vis deblocking conditions is preserved.

In some approaches the same DNA polymerase is used for all steps in the sequencing and rephasing processes. For example, in some cases one polymerase able to recognize and incorporate the nucleotide analog with blocking group-1, and the nucleotide analog with blocking group-2 and/or natural nucleotides is used. Alternatively, a mixture of polymerases with different properties can be used and/or different polymerases with different properties may be used in different steps rather than in the same mixture. In some cases the incorporation step(s) may proceed for 30 sec to 2 min; however, this may vary with the selection of polymerase and other reagents and can be optimized by the practitioner.

Scheme H, shown below, shows that Step 4, as applied to the illustrative clonal population of Scheme G, does not result in incorporation of "C" into any of the growing strands s1-4.

### SCHEME H

| | |
|---|---|
| Target | cgtacgtacgtacgtacgtacgtacgtcacgtacgtacgt (SEQ ID NO: 13) |
| s1 | cgtacgtacgtacgtacgt (SEQ ID NO: 14) |
| s2 | cgtacgtacgtacgtacgt (SEQ ID NO: 14) |
| s3 | cgtacgtacgtacgtacgt (SEQ ID NO: 14) |
| s4 | cgtacgtacgtacgt (SEQ ID NO: 4) |

### Step 5. Repeat Step 2 ("wash").

Steps 1-5 may be referred to as a "Rephasing Round." The rephasing round steps may be repeated multiple times to reduce overall discordance of the numerous different clonal populations on an array or other systems. Three additional rounds are illustrated below. A "re-phrasing module" or "re-phrasing event" (i.e., the totality of steps taken to accomplish substantially complete rephasing at a specified point in the sequencing process) would often consist of 5 to 15 rephasing rounds (e.g. steps 1-5 repeated about 5-15 times). Preferably a rephasing module comprises fewer than 10 rephasing rounds (for example, 4-9, 5-7, 5-9, 6-9, 7-9, 8-9, or 9 rounds). In some cases 10 or 11 rephasing rounds are carried out.

The final step in a rephasing event is de-blocking of Block 2. The de-blocking of Block 2 would only occur once in the rephasing module.

### Round 2

### Repeat Step 1 (Scheme I):

| | |
|---|---|
| Target | cgtacgtacgtacgtacgtacgtacgtcacgtacgtacgt (SEQ ID NO: 13) |
| s1... | cgtacgtacgtacgtacgt**ACGT*** (SEQ ID NO: 15) |
| s2... | cgtacgtacgtacgtacgt**ACGT*** (SEQ ID NO: 15) |
| s3... | cgtacgtacgtacgtacgt**ACGT*** (SEQ ID NO: 15) |
| s4... | cgtacgtacgtacgt**ACGT*** (SEQ ID NO: 6) |

### Repeat Steps 2 and 3 (Scheme J):

| | |
|---|---|
| Target | cgtacgtacgtacgtacgtacgtacgtcacgtacgtacgt (SEQ ID NO: 13) |
| s1... | cgtacgtacgtacgtacgtacgt (SEQ ID NO: 16) |
| s2... | cgtacgtacgtacgtacgtacgt (SEQ ID NO: 16) |
| s3... | cgtacgtacgtacgtacgtacgt (SEQ ID NO: 16) |
| s4... | cgtacgtacgtacgtacgt (SEQ ID NO: 14) |

Repeat Step 4 and Step 5 (Scheme K). In this example C ◄ is not incorporated into any strand in this step.

| | |
|---|---|
| Target... | cgtacgtacgtacgtacgtacgtacgtcacgtacgtacgt (SEQ ID NO: 13) |
| s1... | cgtacgtacgtacgtacgtacgt (SEQ ID NO: 16) |
| s2... | cgtacgtacgtacgtacgtacgt (SEQ ID NO: 16) |
| s3... | cgtacgtacgtacgtacgtacgt (SEQ ID NO: 16) |
| s4... | cgtacgtacgtacgtacgt (SEQ ID NO: 14) |

### ROUND 3

### Repeat Step 1A (Scheme L):

| | |
|---|---|
| Target... | cgtacgtacgtacgtacgtacgtacgtcacgtacgtacgt (SEQ ID NO: 13) |
| s1... | cgtacgtacgtacgtacgtacgt**ACGT*** (SEQ ID NO: 17) |
| s2... | cgtacgtacgtacgtacgtacgt**ACGT*** (SEQ ID NO: 17) |
| s3... | cgtacgtacgtacgtacgtacgt**ACGT*** (SEQ ID NO: 17) |
| s4... | cgtacgtacgtacgtacgt**ACGT*** (SEQ ID NO: 15) |

### Repeat Steps 2 and 3 (Scheme M):

| | |
|---|---|
| Target... | cgtacgtacgtacgtacgtacgtacgtcacgtacgtacgt (SEQ ID NO: 13) |
| s1... | cgtacgtacgtacgtacgtacgtacgt (SEQ ID NO: 18) |
| s2... | cgtacgtacgtacgtacgtacgtacgt (SEQ ID NO: 18) |
| s3... | cgtacgtacgtacgtacgtacgtacgt (SEQ ID NO: 18) |
| s4... | cgtacgtacgtacgtacgtacgt (SEQ ID NO: 16) |

### Repeat Steps 4 and 5 (incorporate C ◄ ) (Scheme N):

| | |
|---|---|
| Target... | cgtacgtacgtacgtacgtacgtacgtcacgtacgtacgt (SEQ ID NO: 13) |
| s1... | cgtacgtacgtacgtacgtacgtacgt**C◄** (SEQ ID NO: 19) |
| s2... | cgtacgtacgtacgtacgtacgtacgtc**C◄** (SEQ ID NO: 19) |
| s3... | cgtacgtacgtacgtacgtacgtacgt**C◄** (SEQ ID NO: 19) |
| s4... | cgtacgtacgtacgtacgtacgt (SEQ ID NO: 16) |

### ROUND 4

### Repeat Step 1 (Scheme O):

| | |
|---|---|
| Target... | cgtacgtacgtacgtacgtacgtacgtcacgtacgtacgt (SEQ ID NO: 13) |
| s1... | cgtacgtacgtacgtacgtacgtacgtC**◄** (SEQ ID NO: 19) |
| s2... | cgtacgtacgtacgtacgtacgtacgtC**◄** (SEQ ID NO: 19) |
| s3... | cgtacgtacgtacgtacgtacgtacgtC**◄** (SEQ ID NO: 19) |
| s4... | cgtacgtacgtacgtacgtacgt**ACGT*** (SEQ ID NO: 17) |

### Repeat Steps 2 and 3 (Scheme P):

| | |
|---|---|
| Target... | cgtacgtacgtacgtacgtacgtacgtcacgtacgtacgt (SEQ ID NO: 13) |
| s1... | cgtacgtacgtacgtacgtacgtacgtc◄ (SEQ ID NO: 19) |
| s2... | cgtacgtacgtacgtacgtacgtacgtc◄ (SEQ ID NO: 19) |
| s3... | cgtacgtacgtacgtacgtacgtacgtc◄ (SEQ ID NO: 19) |
| s4... | cgtacgtacgtacgtacgtacgtacgt (SEQ ID NO: 18) |

### Repeat Step 4 and 5 (Scheme Q):

| | |
|---|---|
| Target... | cgtacgtacgtacgtacgtacgtacgtcacgtacgtacgt (SEQ ID NO: 13) |
| s1... | cgtacgtacgtacgtacgtacgtacgtc◄ (SEQ ID NO: 19) |
| s2... | cgtacgtacgtacgtacgtacgtacgtc◄ (SEQ ID NO: 19) |
| s3... | cgtacgtacgtacgtacgtacgtacgtc◄ (SEQ ID NO: 19) |
| s4... | cgtacgtacgtacgtacgtacgtacgt**C◄** (SEQ ID NO: 19) |

### UNBLOCKING STEP

### Unblock block-2 (Scheme R):

| | |
|---|---|
| Target... | cgtacgtacgtacgtacgtacgtacgtcacgtacgtacgt (SEQ ID NO: 13) |
| s1... | cgtacgtacgtacgtacgtacgtacgtc (SEQ ID NO: 20) |
| s2... | cgtacgtacgtacgtacgtacgtacgtc (SEQ ID NO: 20) |
| s3... | cgtacgtacgtacgtacgtacgtacgtc (SEQ ID NO: 20) |
| s4... | cgtacgtacgtacgtacgtacgtacgtc (SEQ ID NO: 20) |

After removing Block 2 conventional sequencing can be resumed.

### VIII. METHOD 3: DINUCLEOTIDE-FREQUENCY REPHASING (DFR) WITH RUN-FORWARD

Method 3 is similar to Method 2, except that in Step 4, a nucleotide corresponding to the second base of the dinucleotide (C in this example) blocked with Blocking group 2 is added, but nucleotides corresponding to other three nucleotides (e.g. A, G, T), blocked with blocking group-1 are omitted.

### IX. METHOD 4: DINUCLEOTIDE-FREQUENCY REPHASING (DFR) WITH RUN-FORWARD USING BLOCK-1 AND OLIGO BLOCK

In this method, the rephasing position is again a dinucleotide. However, rather than using Block-2 to identify the second nucleotide in the dinucleotide, the second nucleotide is recognized using a degenerate oligonucleotide. The steps of a DFR cycle with oligo block are as follows. For purposes of illustration we assume the starting configuration of the clonal population is as represented in Scheme E, *supra.*

Step 1. Incorporate three natural and 1 reversibly blocked nucleotide with blocking group-1. As an example, the T nucleotide may be the nucleotide comprising blocking group-1, and A, C and G are unblocked (natural nucleotides). The result can be represented as Scheme F, *supra.*

Step 2. Wash to remove polymerase and excess (unincorporated) nucleotides.

Step 3. Remove blocking group 1 from the terminal nucleotides, resulting in a 3' OH group able to accept further nucleotide extension. The result can be represented as Scheme G, *supra.*

### SCHEME G

| | |
|---|---|
| Target | cgtacgtacgtacgtacgtacgtacgtcacgtacgtacgt (SEQ ID NO: 13) |
| s1... | cgtacgtacgtacgtacgt (SEQ ID NO: 14) |
| s2... | cgtacgtacgtacgtacgt (SEQ ID NO: 14) |
| s3... | cgtacgtacgtacgtacgt (SEQ ID NO: 14) |
| s4... | cgtacgtacgtacgt (SEQ ID NO: 4) |

Step 4. Add a 5' phosphorylated oligonucleotide and a ligase (e.g., T4 DNA ligase). Other suitable ligases include, but are not limited to T3 DNA ligase, T7 DNA ligase, Taq DNA ligase as examples.

The 5'-phosphorylated oligonucleotide would typically have a defined base at the 5' position, a degenerate nucleotide composition, and a 3'block. As used in this context, a "degenerate oligonucleotide" may be a pool of oligonucleotides in which multiple bases (sometimes all four bases) occupy many or every position in the oligonucleotide (other then the fixed nucleotide typically at the 5' terminus). Alternatively, or additionally, degenerate bases and/or universal bases may be used at many or every position. Examples of universal bases include 5' nitroindole and deoxylnosine. Alternatively, the 5' end of the oligonucleotide may be pre-adenylated to allow ligase to join the oligonucleotide to the 3' hydroxyl of the terminating strand. 5' phosphorylation may also be achieved by co-reaction with T4 polynucleotide kinase during the ligation reaction to add a 5' phosphate group to a non-phosphorylated oligonucleotide. As indicated the degenerate oligonucleotide is sometimes "partially degenerate" in the sense that some positions may be fixed provided this does not interfer with the function of the olgonucleotide (e.g., annealing to diverse sequences at positions on the template).

As noted, the nucleotide at the 5' position of the oligonucleotide is fixed, and corresponds to the second nucleotide of the selected dinucleotide. A nucleotide at the 5' position of the oligonucleotide "corresponds" to the second nucleotide of the selected dinucleotide if the 5' nucleotide of the oligo and the second nucleotide both can form a base pair with the same base in the template. For example, if the second nucleotide is A, T, G or C, respectively, the 5' nucleotide can be A, T, G or C, respectively. In a preferred embodiment the second nucleotide is T and the 5' nucleotide is Uracil. That is, in one approach, the 5' nucleotide of the oligonucleotide could be a uracil base such that it would base-pair to an A in the template (e.g., DNB) corresponding to a T in the target (reference) sequence. The length of the oligonucleotide is generally in the range of 6 to 12 bases in length or longer. In some embodiments the length is 8, 9, 10, 11 or 12 bases (including the 5' fixed base).

For example, an oligonucleotide of the following general structure may be used:
5'-phos-U(N)z-X ["Degenerate Oligo #1"]
where "5'-phos" is a phosphorylated nucleotide, U is uracil, X is a blocking structure (i.e., a structure that prevents polymerase mediated extension of the oligonucleotide), which may be a non-reversible blocking structure, Z is 6-20, preferably 6-15, more preferably 6-12,and (N)z is a degenerate oligonucleotide sequence. In some examples Z is 9. Examples of non-reversible blocking structures are dideoxy nucleotides and inverted bases (3'-3' linkages offered by oligonucleotide manufacturers, e.g. Integrated DNA Technologies, Coralville, lowa). With increased length of the oligonucleotide the probability of mis-match sequences at the 3' end of the oligonucleotide to the template increases. This, in itself, could inhibit polymerase extension from the 3' end of the blocking oligonucleotide.

Although exemplified by constructs in which uracil is the 5-prime nucleotide in the oligonucleotide and base-pairs with T in the template, in principle cleavage can be carried out by other mechanisms as well, including but not limited to incorporation of ribonucleotides and synthetic nucleotides to create cleavable sites. The method can be carried out using any 5-prime base in which the 5-prime base corresponding to the second nucleotide that allows specific cleavage of the double-stranded molecule immediately 5-prime to the 5-prime base, e.g., when the 5' nucleotide is a cleavage site, such as a removable or abasic nucleotide. In one example, the oligonucleotide is:
5'⁻phos-B(N)_{Z}-X ["Degenerate Oligo #2"]
where B defines a cleavable site. In some cases B is a removable or abasic nucleotide. In some cases B is a a ribonucleotide.

In other embodiments, the cleavable base position of the oligonucleotide does not necessarily have to be at the first position of the oligonucleotide. For example, cleavage at the second position of an oligonucleotide with a degenerate base at the 5-prime position could be applied also. In this example however the first base of the oligonucleotide would become the terminal base of the growing strand but its identity would not be known from the continued sequencing process.

Using Degenerate Oligo #1, if the last base added during the polymerase run-forward steps is a T, as in the illustrative examples discussed above (see, e.g., Scheme F, above, and Scheme AA, below) the dinucleotide recognition sequence will be a TT dinucleotide in the target sequence. The first base (T) is determined by the run-on step and the second base (T) corresponds to the 5' nucleotide of the oligonucleotide (U).

### SCHEME AA

| | |
|---|---|
| Target | cgtacgtacgtacgtacgtacgtacgttcacgtacgtacgt (SEQ ID NO: 21) |
| s1... | cgtacgtacgtacgta**CGT*** (SEQ ID NO: 6) |
| s2... | cgtacgtacgtacgt**ACGT*** (SEQ ID NO: 6) |
| s3... | cgtacgtacgtacgt**ACGT*** (SEQ ID NO: 6) |
| s4... | cgtacgtacgtacg**T*** (SEQ ID NO: 7) |

As illustrated below (Scheme BB), if T is present in the target sequence at the position after polymerase termination (s1-3), then degenerate oligo #1 binds and extension is blocked. if T is not present in the target sequence at the position after polymerase termination (s4), then degenerate oligo #1 would not bind and extension can continue at the next cycle.

### SCHEME BB

| | |
|---|---|
| Target | cgtacgtacgtacgtacgtacgtacgttacgtacgtacgt (SEQ ID NO: 22) |
| s1... | cgtacgtacgtacgtacgtacgtacgt**UNNNNNNNNX** (SEQ ID NO: 23) |
| s2... | cgtacgtacgtacgtacgtacgtacgt**UNNNNNNNNX** (SEQ ID NO: 23) |
| s3... | cgtacgtacgtacgtacgtacgtacgt**UNNNNNNNNX** (SEQ ID NO: 23) |
| s4... | cgtacgtacgtacgtacgtacgt (SEQ ID NO: 16) |

In this method, a re-phrasing module would consist of steps 1-5 repeated about 10 times followed by cleavage of the uracil base. Shown below is the nature of subunits after 3 rounds of extension and ligation (starting from Scheme E)

On each round of extension and ligation each strand will continue to extend and stop at a T if the T is followed by a non-T base. If the following base is also a T then ligation of the 9-mer oligo will proceed with high efficiency, and effectively terminating any further extension.

Shown below is the nature of subunits after 4 rounds of extension and ligation.

### SCHEME CC

| | |
|---|---|
| Target | cgtacgtacgtacgtacgtacgtacgttacgtacgtacgt (SEQ ID NO: 22) |
| s1... | cgtacgtacgtacgtacgtacgtacgt**UNNNNNNNNX** (SEQ ID NO: 23) |
| s2... | cgtacgtacgtacgtacgtacgtacgt**UNNNNNNNNX** (SEQ ID NO: 23) |
| s3... | cgtacgtacgtacgtacgtacgtacgt**UNNNNNNNNX** (SEQ ID NO: 23) |
| s4... | cgtacgtacgtacgtacgtacgtacgt**UNNNNNNNNX** (SEQ ID NO: 23) |

To restart sequencing with a higher number of in-phase subunits, the extended and oligo blocked strands are treated with an enzyme mixture of Uracil-DNA Glycosylase (UDG) and apurinic/apyrimidinic endonuclease 1 (Ape1) or similar abasic site endonuclease. This cleaves and removes the uracil base, leaving a T terminating base with 3'-OH for continuing sequencing. The first sequenced base would be the second T of the TT pair for all DNBs. Other examples of introducing a cleavable site include introducing a single ribonucleotide nucleobase into the oligonucleotide such that after ligation of the oligonucleotide, a single ribonucleotide nucleobase is incorporated surrounded by DNA bases. RNAseHII nuclease, an enzyme specific for such a sequence type can then be use to excise the RNA base with subsequent loss of the 3' side DNA sequences and leaving a 3' DNA terminus for further extension sequencing. For example, in scheme CC rather than a uracil base at the second position, an RNA base is incorporated by virtue of being at the 5' end of the ligating oligo and is subsequently cleaved by RNAseHII. The T base that was the last position of the polymerase extension now becomes the terminal position again for continued strand sequencing extension. Other methods of incorporating a cleavable bond within an oligonucleotide include utilizing a phosphorothiolate bond or "bridging sulfur" linkage with cleavage by silver nitrate (ref PMID: 2027751 Mag et al. Nucleic Acids Res. 1991 Apr 11;19(7):1437-41.)

### X. CUT-BACKS AND OTHER METHODS FOR READJUSTING THE START SITE

Before implementing a rephasing method described above, the user may wish to readjust the starting point of the strand being synthesized in the sequencing reaction backwards or upstream by 5 to 50 bases (a "cutback"). The reason is that in its simplest form, there is no sequence determination during the rephasing. Cutting back or readjusting the position of the strand being synthesized prevents the rephasing from leaving a gap in the sequence, and instead provides the user with a region of sequence overlap to ensure continuity. There are several ways of accomplishing this, as described in the following sections. The cut back process can contribute to the rephasing process in itself but can be limited in that by being restricted to a single nucleotide the probability of some subunits being out of phase is greater than if it was a less frequent sequence event.

### A. READJUSTING THE START SITE OF RUN-FORWARD CYCLES BY INCORPORATING URACIL

One cutback method is to incorporate uracil into the strand being synthesized during the sequencing reaction, and then cleaving at the uracil using an enzyme. In this method, approximately 20 cycles (for example, 5-30 or 10-25 cycles) before starting the phasing resetting module, uracil with a reversible terminator is incorporated in place of reversible terminator T. Sequencing continues for an additional 20 -30 cycles or 20-50 cycles and then the uracil sites are cleaved with an enzyme mixture of Uracil-DNA Glycosylase (UDG) and apurinic/apyrimidinic endonuclease 1 (Ape1) or similar abasic site endonuclease.

The following illustration (Scheme HH) shows the first incorporation step of a uracil reversible terminator. Some subunits will fail to incorporate the uracil either because no "A" is in the template (e.g.DNB) at that particular cycle for that template or clone.

### SCHEME HH

| | |
|---|---|
| Target | cgtacg**t**acgtacgtacgtacg**tc**acgtacgtacgtacgttacgt (SEQ ID NO: 24) |
| s1... | cgtacg**t**a |
| s2... | cgtacg**u** |
| s3... | cgtacg**u** |
| s4... | cgtacg |

Uracil is incorporated for 10 to 20 cycles during the sequencing to ensure most DNBs have incorporated a uracil in the majority of subunits.

### SCHEME II

| | |
|---|---|
| Target | cgtacgtacgtacgtacgtacgtcacgtacgtacgtacgttacgt (SEQ ID NO: 24) |
| s1... | cgtacg**u**acg**u**acguacg**u**acg**u**acgta (SEQ ID NO: 25) |
| s2... | cgtacg**u**acg**u**acguacg**u**acg**u**acgt (SEQ ID NO: 26) |
| s3... | cgtacg**u**acg**u**acguacg**u**acg**u**acgt (SEQ ID NO: 26) |
| s4... | cgtacg**u**acg**u**acguacg**u**acg**u**acg (SEQ ID NO: 27) |

After cleavage of the uracil, some subunits will be in phase while others will not.

### SCHEME JJ

| | |
|---|---|
| ref... | cgtacgtacg**t**acgtacgtacg**tc**acgtacgtacgtacgttacgt (SEQ ID NO: 24) |
| s1... | cgtacg**t**acg (SEQ ID NO: 28) |
| s2... | cgtacg |
| s3... | cgtacg |
| s4... | cgtacg |

After dinucleotide-Frequency Rephasing (DFR) with run-forward using Block-1 and Block-2, the amplicons are configured as follows:

### SCHEME KK

| | |
|---|---|
| ref... | cgtacgtacg**t**acgtacgtacg**tc**acgtacgtacgtacgttacgt (SEQ ID NO: 24) |
| s1... | cgtacg**t**acgTACGTACGTACG**TC** (SEQ ID NO: 29) |
| s2... | cgtacgTACGTACGTACGTACG**TC** (SEQ ID NO: 29) |
| s3... | cgtacgTACGTACGTACGTACG**TC** (SEQ ID NO: 29) |
| s4... | cgtacgTACGTACGTACGTACG**TC** (SEQ ID NO: 29) |

### B. READJUSTING THE START SITE OF RUN-FORWARD CYCLES USING PHOSPHOROTHIOATE NUCLEOTIDES

This method of generating a cut-back of the sequence uses an exonuclease in combination with modified nucleotides that block exonuclease digestion beyond a designated region of the extended sequencing strand.

The first stage of this process is to perform standard sequencing up to a predetermined cycle number. For example, at cycle 200 of a sequencing run the nucleotide incorporation mixture of reversibly terminated nucleotides is switched to one containing a 5' alpha-phosphate thio-modified nucleotide such as 2'-deoxythymidine-(α-thio)-triphosphate. The phosphorothioate bond replaces a non-bridging oxygen at the alpha position phosphate of the tri-phosphate moiety but the nucleotide also possesses the reversible terminator blocking group utilized for the sequencing process. All four bases A, C, G and T would be modified with the (α-thio)-triphosphate.

Sequencing with the (α-thio)-triphosphate nucleotide is allowed to continue for approximately 5 or more cycles and ideally at least 6 cycles. Since mixed isomers of the (α-thio)-triphosphate nucleotide are possible, the ability of the thioate group to block nuclease may be limited to one of the isomer forms. See Yang Z. et al., 2007, Nucleic Acids Res. 35, 3118-3127. By incorporating (α-thio)-triphosphate nucleotides at multiple positions it ensures a high percentage of the strands will be paused during the exonuclease cut-back process at the phosphorothioate modified nucleobases. If a pure preparation of the isomer form that enables nuclease resistance could be guaranteed, then fewer incorporation cycles would be needed.

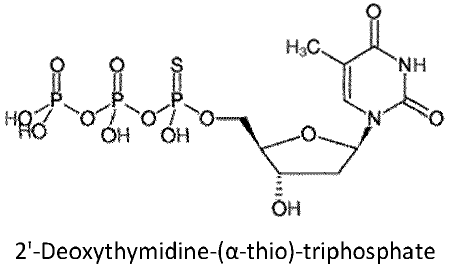

After at least about 6 cycles of incorporation (6-8 cycles), the sequencing mix is then switched back to the standard 5' tri-phosphate nucleotides. Sequencing then continues for a further 30 cycles (30-50 cycles) before initiation of the cut back process. DNA exonuclease (for example Exonuclease III) with specificity towards 3' digestion of a recessed strand in a double stranded structure is then applied to the DNB array to generate a controlled exonuclease reaction to successively degrade the primer strand from the 3' end. See Rogers S. et al., 1980, Methods Enzymol. 65, 201-211. Once the exonuclease process reaches the phosphorothioate bonds the exonuclease reaction is blocked and the cut back process terminates.

Time and reaction conditions are selected to ensure the exonuclease reaction is not extremely excessive to the needed cut back of 30 bases. This minimizes any unwanted side reactions such as the reported ability of exonuclease III to digest single stranded as well as double stranded DNA strands. The initiation of the re-phrasing process can now begin which allows for a run-forward within the 30 base window to ensure no loss of sequence coverage of the target fragment.

### C. READJUSTING THE START SITE OF RUN-FORWARD CYCLES USING A NICKING ENZYME

The start site of run-forward cycles can also be readjusted using a nicking enzyme. The restriction endonuclease Nt.CviPII is a nicking enzyme that recognizes the sequence CCD (where D represents A, G, or T but not C) on double stranded DNA and cleavage occurs on only one strand of the duplex. Nt.CviPII will cleave to the 5' side of the dinucleotide CC on the target DNA.

To target the CC dinucleotide near the terminus of a DNA strand that is being generated during the polymerase based sequencing process and not other CC dinucleotide sequences throughout the double stranded read (sequencing generated strand) and template (e.g., DNB) strands, the enzyme needs to be targeted to the local region of the terminus for example 20-40 bases. This could be achieved by creating a fusion protein between the Nt.CviPII enzyme and antibodies suitable for CooIMPS^{®} sequencing. The CooIMPS^{®} antibodies recognize the terminal incorporated bases by virtue of the 3' blocking group and the base type. A fusion of the Nt.CviPII enzyme and the antibody would create a locally constrained enzyme to the 3' end of the extending strand. Only CCD nucleotides in the extending strand that are within close proximity (closer than 15 or 20 or 25 or 30 or 40 bases) to the 3' end would be targeted.

The DNB strand may be prevented from being targeted by incorporating thiolated bonds during synthesis of the DNB strand or by other methods: for example, constrain on the enzyme-antibody fusion. If both DNB and sequencing strand are thiolated or otherwise modified -- except that in the last 15 to 40 bases of the DNA strand made in sequencing before rephasing cycle, the free in solution nicking enzyme may be used.

To restart sequencing with a higher number of in-phase subunits the extended and Block-2 terminated subunits are cleaved to remove the blocking group, leaving a C terminating base with 3'-OH for continuing sequencing. The first sequenced base would be after the C of the TC pair for all DNBs.

### D. OTHER WAYS OF READJUSTING THE START SITE

An RNA base may be used as an alternative to uracil incorporation. It is likely that a polymerase that contains certain mutations can incorporate RNA bases in addition to accepting reversible terminators. See Gardner et al., 2019, Front Mol Biosci. 2019;6:28. However, the A485L mutation reduces discrimination for rNTPs and allows incorporation of up to twenty ribonucleotides.

Digestion with RNAse HII would then allow cleavage of the extending strand back to the first incorporated RNA base. RNAse HII would allow removal of the RNA containing fragment, leaving a 3'-OH group on the residual DNB hybridized strand that can continue extension. RNA bases also allows use of all four nucleotide bases for incorporation and cleavage. This can be illustrated as shown in FIG. 1.

DNA endonuclease catalyzes the cleavage of the DNA phosphodiester backbone 5' to ribonucleotide, or string of ribonucleotides, embedded within dsDNA, leaving a 3' OH and 5' phosphate.

Another way to remove 15-30 bases from the sequencing strand before forward rephasing is to use controlled 3' exonuclease. One example is Klenow polymerase that removes about 6 nucleotides in one attempt in the reaction without dNTPs. By repeating this process 3-5, 3-7 or 4-7 times the desired number of nucleotides will be removed.

### XI. REVERSIBLE BLOCKING GROUPS

Deoxyribonucleotide analogs with reversible blocking groups are well known in the sequencing arts. Exemplary reversible blocking groups include amino-containing blocking groups (NH₂-).(see Hutter et al., 2010, Nucleosides Nucleotides Nucleic Acids 29(11), allyl-containing blocking group (such as CH₂=CHCH₂-); reversible blocking group comprising a cyano group (such as a cyanoethenyl or cyanoethyl group); azido-containing blocking groups (N₃-), such as azidomethyl (N₃CH₂-); alkoxy-containing blocking group (such as CH₃CH₂O-). In some embodiments, the reversible blocking group contains a polyethylene glycol (PEG) moiety with one or more ethylene glycol units. In some embodiments, the reversible blocking group is a substituted or unsubstituted alkyl, acyl (see, U.S. Patent No. 6,232,465); .methoxymethyl; aminoxyl (H₂NO-); carbonyl (O=CH-); nitrobenzyl (C₆H₄(NO₂)-CH₂-); nitronaphthalenyl; Exemplary groups are described in U.S. Patent No. 10,851,410. In some implementations, nucleotide with a nonremovable (not cleavable) 3' blocking group may be used. In one approach, after detection with an affinity reagent, the last-incorporated base is removed and its position is filed in with a nucleotide that is similar but that has a cleavable blocking group (Koziolkiewicz et al., FEBS Lett. 434:77-82, 1998).

### XII. BLOCKING GROUP CLEAVAGE AGENTS AND CONDITIONS

As discussed above, in some approaches to re-phrasing incorporation of a reversible terminator occurs at the first position of a selected dinucleotide pair, followed by un-blocking cleavage to allow testing of the second position with a reversible terminator that has an alternative 3' blocking group. This allows the continued selective unblocking of the first position until a majority of reads have terminated at the selected dinucleotide pair. There is a general requirement that the un-blocking of the second position should not be facilitated by the un-blocking agent of the first position, but un-blocking of the second position could allow unblocking of the first position. Reversible terminator nucleotide analogs are well known in the art and the practitioner has many options for selecting combinations or pairs of blocking groups with non-overlapping conditions for cleavage suitable for practice of the invention.

In addition to numerous chemical treatments with "non-overlapping" conditions, cleavage using enzymatic conditions, reducing conditions, oxidizing conditions or photo-cleavable conditions would be interchangeable as either position 1 or position 2 un-blocking agents.

A chemical treatment should not significantly degrade the template or primer extension strand. Various molecular moieties have been described for the 3' blocking group of reversible terminators such as a 3'-O-allyl group (Ju et al., Proc. Natl. Acad. Sci. USA 103: 19635-19640, 2006), 3'-O-azidomethyl-dNTPs (Guo et al., Proc. Natl Acad. Sci. USA 105, 9145-9150, 2008), aminoalkoxyl groups (Hutter et al., Nucleosides, Nucleotides and Nucleic Acids, 29:879-895, 2010) and the 3'-O-(2-cyanoethyl) group (Knapp et al., Chem. Eur. J., 17, 2903 - 2915, 2011).

In one example, a reducing agent, such as the phosphine THPP, is used for un-blocking of a first position (eg. o-azidomethyl blocking group) and an oxidizing agent, such as sodium nitrite, for unblocking of a second position aminoxy group (Hutter et al. 2010 Labeled Nucleoside Triphosphates with Reversibly Terminating Aminoalkoxyl Groups. Nucleosides, Nucleotides & Nucleic Acids. 29, 879-895). Blocking moieties with an '-O-allyl group may be cleaved using Pd catalyst generated from Na2PdCl4 and a phosphine ligand P(PhSO3Na)3 (TPPTS) which mediates a deallylation reaction. This allyl could be used as a position 2 blocking group in conjunction with a phosphine cleavable position 1 group such as azidomethyl if the allyl was resistant to phosphine cleavage alone (Ju et al., 2006, Four-color DNA sequencing by synthesis using cleavable fluorescent nucleotide reversible terminators. PNAS. 103, 19635-19640). 3'- O -2-cyanoethyl (CE) group has been reported as a 3' reversible terminator blocking group cleaved with tetrabutylammonium fluoride (TBAF) in THF and small bases like hydroxy groups under alkaline conditions. (Keller et al. Chemlnform Abstract: Synthesis of 3'-O-(2-Cyanoethyl)-2'-deoxythymidine-5'-phosphate as a Model Compound for Evaluation of Cyanoethyl Cleavage. Cheminform. 40 (2009), doi:10.1002/chin.200933204). In a similar way, a cleavable 3' blocking group incorporating a disulfide bond that is cleavable under mild reducing conditions may be suitable as a position 1 blocking group but the position 2 blocking group is such that it requires a stronger reducing agent such as a phosphine for cleavage and or benefits from particular salt and pH conditions for cleavage. Some methoxymethyl 3'-O reversible blocking groups can be cleaved with acid. 3'-O reversible blocking groups that can be cleaved by contacting with an aqueous buffered (pH 5.5) solution of sodium nitrite include, but are not limited to, aminoalkoxyl. Some 3'-O reversible blocking groups can be cleaved by UV light (e.g., nitrobenzyl). Enzymatic cleavage mechanisms may also be used for removal of phosphate blocking groups such as with phosphatases (e.g., shrimp alkaline phosphatase, calf-intestinal phosphatase, antarctic phosphatase and T4 polynucleotide kinase) and esterases (Canard et al., 1995, Catalytic editing properties of DNA polymerases. Proc Natl Acad Sci USA. 92, 10859-10863). Photo-cleavable blocking groups such 3'-o-nitrobenzyl have also been described that would be compatible with chemical or enzymatic cleavage methods (Metzker et al. 1994. Termination of DNA synthesis by novel 3'-modified-deoxyribonucleoside 5'-triphosphates. Nucleic Acids Res. 22, 4259-4267 (1994)).

### XIII. ADDITIONAL EMBODIMENTS

The discussion above describes, *inter alia,* methods for rephasing to a specified dinucleotide. The reader guided by this disclosure will recognize that rephasing can be designed to target a 3-base sequence (trinucleotide). In one aspect, for example, the invention provides a method of rephasing extended primers in a clonal population of nucleic acid duplexes comprising extended primers hybridized to a template sequence, wherein a plurality of the extended primers in the clonal population have different 3' ends and are thereby out of phase, the method comprising extending the extended primers by incorporating nucleotides that are complementary to the template sequence using a polymerase and nucleotides comprising nucleotide triphosphates A, T, C, and G, or analogs thereof, to the first target sequence 1-3 (e.g., 3) nucleotides in length until substantially all of the extended primers reach the target sequence. Stopping at a trinucleotide sequence involves first stopping at dinucleotides (as described in detail above)removing the second block, and continuing process using the same or a new nucleotide or multiple nucleotides (for multiple different 3-mers being selected for stopping) having second block. For example, after stopping at CA dinucleotides, one can we continue with 5 cycles of extensions with A having the second blocking group and other three nucleotides having the first blocking group that is cleaved after each of 5 cycle. In this example, extension would stop at these 3-mers: CA(noA)₀₋₅A. If second blocking group is used for T and C, extension would stop at both CA(noT)₀₋₅T and CA(noC)₀₋₅C trinucleotides. In one approach the 3mer(s) is selected based on frequency in the sequence, and is tuned to stop extension approximately every 10, every 20, every 25, or every 30 bases.

In one approach the invention provides a method of rephasing extended primers in a clonal population of nucleic acid duplexes comprising extended primers hybridized to a template sequence, wherein a plurality of the extended primers in the clonal population have different 3' ends and are thereby out of phase, the method comprising cutting the extended primers to a furthest target sequence 1-4 bases in length within a predefined window of 20, or 30, or 40, bases. Cutting may be used to rephase 3' or 5' ends or both strands (cutting both strands at the target sequence). 5-primer cutting can utilize a nicking enzyme with a known recognition sequence that may be attached to 5' end with a linker that defines how far the enzyme can cut. To illustrate
- copy1: ...BBBBCC'BBBBBBBBB-5'-linker-NE that nicks after CC ...BBBBGGBBBBBBBBBBBBBBB-3'
- copy2: ...BBBBCC'BBBBBBB-5'-linker NE that nicks after CC ...BBBBGGBBBBBBBBBBBBBB-3'
NE means nicking enzyme. The linker may have a length that allows the nicking enzyme to cleave at sites within 10 bases. Both copies would be rephased at the CC even when copy1 has 9 bases from CC to 5' end and copy2 has 7 bases from cc to 5' end.

Although embodiments in this disclosure are generally presented in the context of SBS sequencing it is contemplated that the methods described herein may be used for several purposes, including but not limited to a group of ends generated by a wobbling restriction enzyme or incompletely synchronized primer extension or exonuclease degradation, or any other uses in which members of a clonal population are not in phase.

It will be recognized by the reader guided by the specification that trivial changes can be made relative to the description above, all of which are contemplated by the inventors. The following hypothetical example is provided.

| Nucleotide | First scheme | Alternative scheme |
|---|---|---|
| A | A* | A* |
| T | T◄ | T◄ |
| G | G◄ | G▲ |
| C | C◄ | C▼ |
| | * is second blocking group | * is second blocking group |
| | ◄ is first blocking group | ◄ is first blocking group |
| | | ▲ is first blocking group |
| | | ▼ is first blocking group |

In this hypothetical a first scheme is shown with nucleotides blocked with a first blocking group and a second blocking group. The second scheme shows nucleotides blocked with a first blocking group, a second blocking group, a third blocking group, and a fourth blocking group, where the third and fourth blocking groups are equivalents to or variants of the first blocking group and have the same properties in relation to the second blocking group. It will be understood that reference to "a blocking group" for example encompasses functional equivalents (i.e., a first blocking group or functional equivalents that share a property with the first blocking group).

### XIV. POLYMERASES

Any DNA polymerase used in sequencing may be used in the methods disclosed herein, including, for example, a DNA polymerase from Thermococcus sp., such as 9° N or mutants thereof, including A485L, including double mutant Y409V and A485L. Exemplary DNA polymerases and methods that may be used include those described in Chen, C., 2014, "DNA Polymerases Drive DNA Sequencing-By-Synthesis Technologies: Both Past and Present" Frontiers in Microbiology, Vol. 5, Article 305, Pinheiro, V. et al. 2012 "Polymerase Engineering: From PCR and Sequencing to Synthetic Biology" Protein Engineering Handbook: Volume 3:279-302. International patent publications WO 2005/024010 and WO 2006/120433. In some cases the polymerase is DNA polymerase from Thermococcus sp., such as 9° N or mutants thereof, including A485L, including double mutant Y409V and A485L. Other examples include E. coli DNA polymerase I, Klenow fragment of DNA polymerase I, T7 or T5 bacteriophage DNA polymerase, HIV reverse transcriptase; Phi29 polymerase, and Bst DNA polymerase.

### XV. KITS

This disclosure provides kits or reagent combinations for use in rephasing. A kit comprised two reversible terminator nucleotide triphosphates, where each has a different reversible blocking group, and the two reversible blocking groups are removable under different conditions is described.

For dinucleotide rephasing, the kit may contain the first nucleotide triphosphate (selected from A, T, C, or G) blocked with a first reversible blocking group, and a second nucleotide triphosphate (independently selected from A, T, C, or G) blocked with a second reversible blocking group that is different from the first blocking group and removable under different conditions. Possible blocking groups include but are not limited to those listed above in Section XV. Exemplary is a first blocking group of O-azidomethyl, and a second blocking group comprising a ONH2 group. The kit may also contain chemical reagents suitable for removing each of the blocking groups at an appropriate time during rephasing.

Alternatively, a kit for dinucleotide rephasing may contain a first nucleotide triphosphate (one to four selected from A, T, C, or G) blocked with a first reversible blocking group, and an oligonucleotide configured to hybridize to the template adjacent to the growing primer when the second nucleotide is present, typically at the 5' end of the oligonucleotide, optionally accompanied with a ligase suitable for ligating the oligonucleotide to the 3' end of the sequencing primer. Other positions of the oligonucleotide may be degenerate and/or universal bases, as explained earlier. The oligonucleotide is typically blocked at the 3' end, but is removable from the growing primer after the dinucleotide selected for rephasing has been encountered. A kit containing such an oligonucleotide may also contain a reagent for removing the first blocking group, and one or more reagents for removing the oligonucleotide, such as an enzyme mixture of Uracil-DNA Glycosylase (UDG) and Apurimac/apyrimidinic endonuclease 1 (Ape1) or similar abasic site endonuclease.

In some embodiments the oligonucleotide has the following structure: 5'-Phos-U(N)_{Z}-X where "Phos" indicates the oligonucleotide is 5' phosphorylated, "U" is uracil, Z is 6-20, preferably 6-12, preferably 9, "(N)z" is a sequence of Z degenerate bases; and "X" is a non-reversible blocking structure (including, without limitation, a dideoxy nucleotide or inverted base).

Any of these kits may further contain one or more reagents for use in repositioning the growing strand by way of a cutback: for example, a uracil triphosphate (with or without UDG and Ape1), nucleotides with an RNA or thiolated base, and/or an endonuclease or an exonuclease.

More comprehensive kits include any of these reagent combinations for rephasing and/or repositioning extending sequencing primers, in combination with other reagents used for sequencing by synthesis: for example, reversible terminators with directly attached fluorophores or labeled antibodies, a DNA polymerase, and reagents for preparing concatemers, DNB arrays, bridge PCR strands, and other clonal populations of DNA fragments to be sequenced.

Reagents in such kits are generally supplied separately or in working mixtures in standard containers or in modules that are specialized for drawing the reagents into a sequencing apparatus or a flow cell. The reagents are optionally accompanied by or distributed in combination with instruction for use of the reagents in sequencing and rephasing in accordance with this disclosure.

### XVI. IN SILICO REPHASING

The technology put forth in this disclosure was modelled *in silica* to demonstrate its effectiveness in reducing discordance of the growing strand and increasing effective read length. Amongst the choices for rephasing outlined above, the process used in this simulation comprised the following:
(1) a cutback of 30 bases, such as can be done by introducing uracil into the growing strand 30 cycles back in the sequencing, and cleaving with UDG/Ape1 enzyme mixture, corresponding to an A base in the sequence;
(2) a rephasing event using the dinucleotide-frequency rephasing process described above as "Method Two". Simulations were done using the alternative dinucleotides CA or CG. Each rephasing cycle comprised extending non-blocked strands to the first base in the pattern (C), and then if the next base was the second base in the rephasing dinucleotide (A or G), then a blocker is inserted. This can be illustrated as follows:

| | |
|---|---|
| | AACTACAGCTGC (SEQ ID NO: 30) - original starting position |
| New P: | AACTACAGCTGC (SEQ ID NO: 30) - read position moved ~ 10 bases back |
| Step 1: | AACTACAGCTGC (SEQ ID NO: 30) - extends to the first C, next base not A |
| Step 2: | AACTACAGCTGC (SEQ ID NO: 30) - extends to the next C, adds blocker to A |
| Step 3: | AACTACAGCTGC (SEQ ID NO: 30) - stays at A due to the blocker |

### A. Prophetic reaction conditions

In practical terms, the underlying technology for the *in silico* experiment can be implemented as follows. DNA nanoballs (DNBs) (concatemers of nucleic acid templates to be sequenced) are arrayed on a solid surface, and analyzed by sequencing by synthesis, determining each base sequentially guided by a complementary strand.

DNBs are generated by amplification of concatemers to create single stranded multiverse of a reverse complement single stranded circle as described previously (R. Drmanac et al., Science. 327, 78-81, 2010). The DNB arrays are sequenced by the step-wise addition of 3' blocked reversibly terminated nucleotides with a DNA polymerase, followed by detection with fluorescently labeled antibodies ("CoolMPS", U.S. Patent No. 10,851,410). The 3' reversible terminator group is o-azidomethyl (AzM) which is unblocked with 10 mM Tris(hydroxypropyl) phosphine (THPP) for 2 minutes at 55ºC. Cleavage of the 3' blocking group to a 3' hydroxyl group allows continued incorporation for sequential base determination.

### B. Error simulation

To perform the computer simulation, sequencing was assumed to continue in multiple cycles of sequencing-by-synthesis with computer-generated errors entered into the data in each sequencing cycle at about the same frequency known to occur in live flow cell sequencing. The effect of rephasing was determined assuming the cutback and rephasing events went to completion.

The simulation parameters were as follows: DNB Count: 977029, copy number (fragment copies per DNB): 180 (CV: 20%). Two target sequences were used: one, a portion of a human genomic DNA reference sequence; the second, a computer generated random sequence having about the same overall composition: A and T, 27%; C and G, 23%. The sequencing simulation samples regions from reference genomes and models the phasing and labelling stochastics for an array of incorporation sites corresponding to independent copies on each DNB. The labeled sites are aggregated into the respective channels based on the sequence context of the sites position. This generates an array containing the number of labelled sites present for each channel at each cycle.

The sequencing errors introduced were 0.1% lag, 0.05% run-on, and 0.15% termination. The lag and run-on cause the copy being sequenced to go out of phase, whereas the termination halts further chemistry on that copy, effectively causing a decrease in intensity from the host DNB.

Either 5 or 7 rephasing cycles were simulated for each rephasing event. Two rephasing events were done after 300 or 600 bases of the sequencing, or three rephasing events were done after 225, 450, and 675 bases of sequencing.

The output data is simulated based on a fluorescent label detected in each cycle for each primer in each DNA nanoball arranged in a grid pattern. The grid can be adjusted for different distances between DNBs as well as different pixel resolutions. A normal distribution of pixel values was added to the image to simulate the effect of background. The results shown are based on distancing, distribution, and background that is typical of DNA sequence devices used for nanoball sequencing.

### C. Cutback step using uracils

Since the run-forward incorporation during rephasing creates a gap in sequencing data under these conditions, a cut-back process is first used to remove a section of DNA already sequenced, to ensure no loss of sequence coverage. The computer model assumes a cutback of 30 residues before each rephasing event.

In actual practice, this can be done as follows. Thirty cycles before the sequencing by synthesis is paused, the standard sequencing-by-synthesis reagent mix is switched to one containing 3 µM each of dUTP-AzM, dATP-AzM, dCTP-AzM, dGTP-AzM and the DNA polymerase. Because of this switch, dUTP-AzM replaces dTTP-AzM and sequencing continues for a further 30 cycles with the alternate incorporation mix. The antibody normally used for recognition of the dTTP-AzM nucleotide can be used during the dUTP containing cycles as well, providing that it has sufficient cross-reactivity and specificity to recognize the dUTP-AzM nucleotide.

After the final 30 cycles of sequencing, the extended primers are treated with Uracil-DNA Glycosylase (UDG) enzyme (2 U/µL) and Apurinic/apyrimidinic Endonuclease 1 (Ape1) endonuclease enzyme (1 U/µL) mixture for 10 min at 37ºC to cleave uracil bases and the subsequently generated abasic sites. The effect of this is to cut- back the extended and sequenced DNA strand to the first uracil incorporated of the 30 cycles of incorporation utilizing dUTP-AzM. After buffer exchange to remove the UDG/Ape1 mixture, the flow cell is washed multiple times at 55ºC in low salt buffer to remove short cleavage sequences.

### D. Dinucleotide rephasing step

After the cutback, the computer model assumes that the primers will be extended again until the selected dinucleotide is recognized and blocked.

In actual practice, this can be done as outlined in Table 1.

| TABLE 1: Process steps for a dinucleotide re-phrasing of a DNB arrayed flow cell | | | |
|---|---|---|---|
| Step | Process | Temp | Time |
| Step 1A | Incorporate dCTP-AzM, dATP, dGTP, dTTP | 55ºC | 2 min |
| | AzM de-block | 55ºC | 2 min |
| Step 1B | Incorporate dCTP-AzM, dATP-ONH2, dGTP-AzM, dTTP-AzM | 55ºC | 2 min |
| | AzM de-block | 55ºC | 2 min |
| Step 2A | Incorporate dCTP-AzM, dATP, dGTP, dTTP | 55ºC | 2 min |
| | AzM de-block | 55ºC | 2 min |
| Step 2B | Incorporate dCTP-AzM, dATP-ONH2, dGTP-AzM, dTTP-AzM | 55ºC | 2 min |
| | AzM de-block | 55ºC | 2 min |
| Step 3A | Incorporate dCTP-AzM, dATP, dGTP, dTTP | 55ºC | 2 min |
| | AzM de-block | 55ºC | 2 min |

| TABLE 1: Process steps for a dinucleotide re-phrasing of a DNB arrayed flow cell | | | |
|---|---|---|---|
| Step | Process | Temp | Time |
| Step 3B | Incorporate dCTP-AzM, dATP-ONH2, dGTP-AzM, dTTP-AzM | 55ºC | 2 min |
| | AzM de-block | 55ºC | 2 min |
| Step 4A | Incorporate dCTP-AzM, dATP, dGTP, dTTP | 55ºC | 2 min |
| | AzM de-block | 55ºC | 2 min |
| Step 4B | Incorporate dCTP-AzM, dATP-ONH2, dGTP-AzM, dTTP-AzM | 55ºC | 2 min |
| | AzM de-block | 55ºC | 2 min |
| Step 5A | Incorporate dCTP-AzM, dATP, dGTP, dTTP | 55ºC | 2 min |
| | AzM de-block | 55ºC | 2 min |
| Step 5B | Incorporate dCTP-AzM, dATP-ONH2, dGTP-AzM, dTTP-AzM | 55ºC | 2 min |
| | AzM de-block | 55ºC | 2 min |
| Step 6 | ONH2 de-block | 25ºC | 2 min |
| AzM: 3' o-azidomethyl blocking group, ONH2: 3'-aminoxy modified dATP, Firebird Biomolecular Sciences, LLC 3'-aminoxy blocking group. | | | |

An incorporation mix is created consisting of 1 reversible terminator dCTP-AzM, and three non-blocked natural nucleotides dATP, dGTP and dTTP. Typically, the nucleotides are included at a concentration of 3 µM, accompanied with a DNA polymerase: for example, a DNA polymerase variant able to incorporate both the azido methyl 3' blocking group and the natural nucleotide (U.S. Patent No. 10,851,410). The time of incorporation (step 1A) is typically 2 min, and occurs at a temperature of 55ºC. Natural nucleotides are free to incorporate in a sequential fashion as dictated by the template sequence but upon incorporation of a dCTP-AzM, extension ceases because of the 3' blocking group. After the first incorporation step, cleavage of the incorporated dCTP-AzM group occurs by incubation with THPP at a concentration of 10 mM for 2 min. Again, the temperature of the reaction is maintained at 55ºC.

Cleavage results in conversion of the 3' blocking group to 3' hydroxyl which allows further incorporation to the terminal cytosine. The second incorporation reaction (Step 1B) consists of 3' aminoxy modified dATP (dATP-ONH2) as a blocking nucleotide, dCTP-AzM, dGTP-AzM, dTTP-AzM nucleotides, and the 9°N variant DNA polymerase. A second cleavage occurs with 10 mM THPP to specifically unblock the incorporated C, G and T nucleotides for further extension. Those terminal C bases that incorporated a dATP-ONH2 as the next base are resistant to cleavage by THPP and so stay blocked to further extension.

To complete a rephasing event, Steps 1A and 1B are repeated a further four times. Step 6 is the unblocking of all incorporated and accumulated dATP-ONH2 nucleotides with 700 mM Sodium Nitrite at 25°C for 2 min. Since the majority of copies of DNBs are now in phase sequencing determination can start again.

### E. Results of the simulation

Implementing into the computer model the parameters and error variables referred to in subsection "B", above, the results were as follows.

FIG. 2 shows the percentage of DNB templates in which 100% of the copies or subunits were rephased back to the reference sequence, compared amongst different rephasing conditions. Each triplet shows the extent of rephasing after the first, second or third rephasing event. Using CA as the rephasing dinucleotide was somewhat more effective than using CG; Seven (7) rephasing cycles was somewhat more effective than 5; three rephasing events was somewhat more effective than two. The difference between the CA dinucleotide and the CG dinucleotide for the human sequence is more pronounced, because CG occurs less frequently than CA does in the human genome. CA and CG do not occur in exact equal frequency for the randomly simulated sequences (CA: approximately 6.21% vs CG: approximately 5.29%), although they are significantly closer in frequency compared with the Human reference (CA: approximately 7.27% vs CG: approximately 0.99%).

It is remarkable that all conditions resulted in a rephasing of over 85% of the DNBs. Without any rephasing at all, the number of DNBs in phase is close to zero. Out of roughly *a million* DNBs on the array, there are *only two* DNBs that have more than 95% of sites in phase at the various rephasing cycles. At sequencing cycle 225, one DNB has one site in the minus one position and 35 sites in phase. At sequencing cycle 675, one DNB has only a single active site that is in phase.

FIG 3 shows the synchronized percentage of DNBs. This data explores a cause for the increasing percentage of DNBs being 100% in phase between rephasing events shown in FIG 2. One explanation for this trend is that the stochastics of termination leads to the elimination of out of phase sites for DNBs that did not fully synchronize in previous rephasing events. This would cause DNBs that were previously not 100% in phase by only a couple sites to transition having a higher probability of being fully in phase during the next round of rephasing. The data in FIG. 3 illustrate how the percentage of DNBs that only have one site out of phase is the reverse of the percentage of DNBs that are fully in phase. While the percentage of fully synchronized DNBs increases between rephasing events, the percentage of DNBs with only one site out of phase decreases. When the two percentages are added together, the percentage of DNBs <= 1 site out of phase remains consistent between rephasing events.

Statistics for the different rephasing conditions are compared in Table 2. The statistics shown in the table as bold and/or underlined are somewhat superior. All conditions were effective.

FIG. 4A compares the kinetics of phase discordance of the growing strand between the different parameters tested on the human reference sequence. FIG. 4B is a similar comparison for the randomly generated reference sequence. Under the conditions of the simulation, without rephasing, the discordance accumulates rapidly after the 300^{th} sequencing cycle, and is over 5% at the 500^{th} cycle. Two rephasing events keeps the discordance below 2% for over 750 cycles. Three rephasing events keeps the discordance below 2% for over 900 cycles. The extent of discordance is also shown in TABLE 3.

FIG. 5 shows the cumulative cycle offset after the final rephasing event. This is a survival curve, with the X-axis corresponding to the cumulative cycle offset that is expected after the last rephasing event. A negative value would correspond to the generation of overlapping sequence regions during rephasing, while a positive value would correspond to sequencing past the allotted number of cycles. In the best-case scenarios the CG dimer pattern will result in 40% of the DNBs sequencing past the end point, compared to only about 8% of DNBs for the CA dimer pattern. These curves illustrate that there may be a need to have a small buffer region past the end of sequencer cycles to prevent a significant number of DNBs from sequencing into the adapter (for example: 950 bases for 900 cycles of sequencing).

Clearly, the dimer pattern used for rephasing played a pivotal role in the percentage of DNBs that will eventually sequence past the allotted number of bases. This can both affect the number of DNBs that sequence into the adapter region, as well as the length of overlapping sequences generated during the rephasing process.

The invention has been described in this disclosure with reference to the specific examples and illustrations. The features of these examples and illustrations do not limit the practice of the claimed invention, unless explicitly stated or otherwise required.

.

**TABLE 2: Statistics comparison for the rephasing simulation**

| Ref | cut back window | phasing cycles | doublet | events | doublet sync DNB% | doublet sync 100% In Phase | first base DNB% | first base 100% in phase | total DNB% 100% in phase | off strand DNB% |
|---|---|---|---|---|---|---|---|---|---|---|
| human | 30 | 7 | CG | 3 | 25.69% | 94.82% | 74.28% | 84.93% | 87.45% | 0.071% |
| human | 30 | 7 | CA | 3 | 94.40% | 94.33% | 5.59% | 83.74% | 93.72% | 0.021% |
| human | 30 | 5 | CG | 3 | 19.63% | 94.10% | 80.35% | 84.74% | 86.56% | 0.05% |
| human | 30 | 5 | CA | 3 | 38.22% | 93.96% | 11.78% | 84.86% | 92.88% | 0.019% |
| human | 30 | 5 | CG | 2 | 19.53% | 94.09% | 80.46% | 84.10% | 86.04% | 0.018% |
| human | 30 | 5 | CA | 2 | 83.23% | 93.84% | 11.72% | 83.96% | 92.68% | 0.008% |
| random | 30 | 7 | CG | 3 | 83.95% | 94.85% | 16.05% | 85.19% | 93.30% | 0% |
| random | 30 | 7 | CA | 3 | 91.44% | 94.48% | 8.56% | 85.70% | 93.73% | 0% |
| random | 30 | 5 | CG | 3 | 72.93% | 94.29% | 27.07% | 84.83% | 91.73% | 0% |
| random | 30 | 5 | CA | 3 | 82.76% | 93.94% | 17.24% | 85.61% | 92.51% | 0% |
| random | 30 | 5 | CG | 2 | 73.00% | 94.22% | 27.00% | 84.00% | 91.46% | 0% |
| random | 30 | 5 | CA | 2 | 82.71% | 93.86% | 17.29% | 84.81% | 92.29% | 0% |

**TABLE 3: Discordance comparison for the rephasing simulation**

| Ref. | step # | doublet | events | average | C0-300 | C300-600 | C600-900 |
|---|---|---|---|---|---|---|---|
| human | 7 | CA | 3 | 0.38 | 0.003 | 0.114 | 1.033 |
| human | 7 | CG | 3 | 0.37 | 0.005 | 0.128 | 0.989 |
| human | 5 | CA | 3 | 0.42 | 0.003 | 0.123 | 1.125 |
| human | 5 | CG | 3 | 0.40 | 0.005 | 0.136 | 1.058 |
| human | 5 | CA | 2 | 0.77 | 0.012 | 0.353 | 1.936 |
| human | 5 | CG | 2 | 0.74 | 0.013 | 0.386 | 1.812 |
| human | * | * | 0 | 8.18 | 0.013 | 3.229 | 21.322 |
| human | 7 | CA | 3 | 0.33 | 0.004 | 0.102 | 0.885 |
| human | 7 | CG | 3 | 0.36 | 0.004 | 0.116 | 0.949 |
| human | 5 | CA | 3 | 0.38 | 0.004 | 0.119 | 1.011 |
| human | 5 | CG | 3 | 0.39 | 0.004 | 0.130 | 1.050 |
| human | 5 | CA | 2 | 0.70 | 0.012 | 0.344 | 7.757 |
| human | 5 | CG | 2 | 0.73 | 0.012 | 0.367 | 1.809 |
| human | * | * | 0 | 8.28 | 0.012 | 3.102 | 21.740 |

| | | | | | | | |
|---|---|---|---|---|---|---|---|
| (*) = no rephasing | | | | | | | |

## Claims

1. A method of rephasing extended primers in a clonal population of nucleic acid duplexes comprising extended primers hybridized to a template sequence, wherein a plurality of the extended primers in the clonal population have different 3' ends and are thereby out of phase, the method comprising:
(1) extending the extended primers by incorporating one or more nucleotides that are complementary to the template sequence using a polymerase and nucleotides comprising nucleotide triphosphates A, T, C, and G, or analogs thereof, wherein one of the nucleotides is a reversible terminator blocked with a first blocking group and the other three nucleotides are not blocked, until substantially all of the extended primers are blocked; and then
(2) unblocking the extended primers,
wherein the rephasing comprises dinucleotide-frequency rephasing (DFR), in which each extended primer is extended until a selected dinucleotide XY is reached.

2. The method of rephasing according to claim 1,
wherein the first nucleotide (X) is the reversible terminator blocked with the first blocking group, and the second nucleotide (Y) is a reversible terminator blocked with a second blocking group.

3. The method of claim 2 comprising:
(a) performing multiple cycles of the following:
(i) further extending the extended primers using a first mixture that contains a polymerase and four nucleotide triphosphates selected from A, T, C, and G and/or analogs thereof, wherein one of the nucleotide triphosphates or analogs in the first mixture corresponds to the first nucleotide (X) of the selected dinucleotide and is blocked with a first blocking group, and wherein the other three nucleotide triphosphates or analogs in the first mixture are unblocked, the extending being continued until substantially all of the extended primers are blocked with the first blocking group; then
(ii) unblocking the first blocking group; and
(iii) treating the extended primers from (ii) with a second mixture that contains a polymerase and a single nucleotide triphosphate selected from A, T, C, or G and analogs thereof that corresponds to the second nucleotide (Y) of the selected dinucleotide and is blocked with a second blocking group, wherein the second mixture optionally includes the three nucleotide triphosphates or analogs not corresponding to the second nucleotide (Y) blocked with the first blocking group,
(b) repeating step (a) until substantially all of the extended primers are blocked with the second blocking group; and
(c) unblocking the second blocking group;
thereby rephasing the extended primers in the clonal population.

4. The method of claim 3, wherein either
(A) the only nucleotide triphosphate in the second mixture is the nucleotide triphosphate or analog that is blocked by the second blocking group; or,
(B) the second mixture contains the nucleotide triphosphate or analog blocked by the second group, and the three nucleotide triphosphates or analogs not corresponding to the second nucleotide (Y) blocked with the first blocking group.

5. The method of any of claims 2 to 4, wherein either of the first and second blocking groups is an O-azidomethyl group, and the other of the first and second blocking groups is an O-NH₂ group.

6. The method of claim 2 comprising:
(a) performing multiple cycles of the following:
(i) further extending the extended primers using a first mixture that contains a polymerase and four nucleotide triphosphates selected from A, T, C, and G and/or analogs thereof, wherein one of the nucleotide triphosphates or analogs in the first mixture corresponds to the first nucleotide (X) of the selected dinucleotide and is blocked with a first blocking group, and wherein the other three nucleotide triphosphates or analogs in the first mixture are unblocked, the extending being continued until substantially all of the extended primers are blocked with the first blocking group; then
(ii) unblocking the first blocking group; and
(iii) treating the extended primers with a second mixture that contains a ligase and a 5' phosphorylated oligonucleotide blocked at the 3' end, wherein a base in the oligonucleotide corresponds to the second nucleotide (Y) of the selected dinucleotide;
(b) repeating step (a) until substantially all of the extended primers are blocked with the oligonucleotide; and
(c) unblocking the oligonucleotide;
thereby rephasing the extended primers in the clonal population.

7. The method of claim 6, wherein the 5' phosphorylated oligonucleotide has the formula A(N)₁₋₁₅-B, wherein A is a nucleotide base that corresponds to the second nucleotide (Y) of the selected dinucleotide, each N is a nucleotide homolog or a nucleotide mixture containing a nucleotide that can hybridize to any base in the template sequence; and B is a non-reversible blocking structure; and wherein the unblocking in step (c) comprises removing the oligonucleotide from the extended primer.

8. The method of claim 7, wherein the non-reversible blocking structure is inverted dT (IDT) incorporated at the 3'-end of the oligonucleotide, thereby creating a 3'-3' linkage which inhibits both degradation by 3' exonucleases and extension by DNA polymerases.

9. The method of claim 7, wherein A is uracil, and wherein 5' phosphorylated oligonucleotide is unblocked by treating with an enzyme mixture of uracil-DNA glycosylase (UDG) and apurinic/apyrimidinic endonuclease 1 (Ape1) to cleave and remove the uracil base.

10. The method of any of claims 3 to 9, wherein five to fifteen cycles are performed in step (a).

11. The method of any of claims 1 to 10, wherein five to fifty bases are removed from the 3' end of each primer before the rephasing, thereby readjusting the 3' end of the extended primers to an upstream position.

12. The method of claim 11, wherein the readjusting comprises:
(i) during sequencing-by-synthesis done before the rephasing, including in at least some of the cycles of the sequencing a uracil triphosphate or analog thereof that can be incorporated into the extended primer in place of thymine triphosphate; then
(ii) cleaving the extended primers at incorporated uracil bases.

13. The method of claim 12, wherein the cleaving in step (ii) is done using an enzyme mixture of uracil-DNA glycosylase (UDG) and apurinic/apyrimidinic endonuclease 1 (Ape1).

14. The method of claim 11, wherein the readjusting comprises:
(i) during sequencing-by-synthesis done before the rephasing, including in at least some of the cycles of the sequencing a nucleotide triphosphate that contains an ribonucleotide (RNA) or a 5' alpha-phosphate thio-modified nucleotide; then
(ii) cleaving the extended primers at incorporated RNA bases or at incorporated 5' alpha-phosphate thio-modified nucleotides.

15. The method of claim 11, wherein the readjusting comprises treating the extended primers with a 3' exonuclease under controlled conditions, or treating the extended primers with a nicking enzyme that is sub-sequence dependent, thereby removing said five to fifty bases from the 3' end of the extended primer.

16. The method of any of claims 1 to 15, further comprising resuming cycles of sequencing after the rephasing, whereby the extended primers in the clonal population are extended by bases that each identify a complementary nucleotide in the template sequence.

17. A method of obtaining long sequencing reads from a clonal population of nucleic acid duplexes each comprising an extended primer annealed to a template sequence, the method comprising:
performing multiple cycles of sequencing in which the extended primer in each duplex is extended by one nucleotide, thereby identifying a complementary nucleotide in the template sequence;
after a number of such sequencing cycles, rephasing the extended primers according to the method of any of claims 1 to 16; then
resuming cycles of the sequencing to identify further nucleotides in the template sequence.

18. The method of claim 17, wherein the rephasing is done two to four times within the first 800 sequencing cycles.

19. The method of claim 17 or claim 18, wherein the rephasing extends the number of clonal populations having a discordance percentage of less than 2% by at least 1.5-fold; or,
wherein the rephasing extends the number of clonal populations having a discordance percentage of less than 2% by at least 200 cycles.

20. The method of any of claims 1-19, wherein each clonal population on the array is a DNA nanoball or concatemer; or
wherein each clonal population is a cluster of DNA strands produced by bridge polymerase chain reaction (PCR) or copies of a template sequence in an emulsion droplet.

## Patentansprüche

1. Verfahren zum Neuphasieren von erweiterten Primern in einer klonalen Population von Nukleinsäureduplexen, die erweiterte Primer umfassen, die an eine Vorlagensequenz hybridisiert sind, wobei eine Vielzahl der erweiterten Primer in der klonalen Population unterschiedliche 3'-Enden aufweist und dadurch phasenverschoben ist, wobei das Verfahren Folgendes umfasst:
(1) Erweitern der erweiterten Primer durch Einbauen von einem oder mehreren Nukleotiden, die komplementär zu der Vorlagensequenz sind, unter Verwendung einer Polymerase und von Nukleotiden, die Nukleotidtriphosphate A, T, C und G oder Analoga davon umfassen, wobei eines der Nukleotide ein reversibler Terminator ist, der mit einer ersten Blockierungsgruppe blockiert ist, und die anderen drei Nukleotide nicht blockiert sind, bis im Wesentlichen alle der erweiterten Primer blockiert sind; und dann
(2) Entblocken der erweiterten Primer, wobei das Neuphasieren Dinukleotid-Frequenz-Neuphasieren (DFR) umfasst, bei dem jeder erweiterte Primer erweitert wird, bis ein ausgewähltes Dinukleotid XY erreicht ist.

2. Verfahren zum Neuphasieren gemäß Anspruch 1,
wobei das erste Nukleotid (X) der reversible Terminator ist, der mit der ersten Blockierungsgruppe blockiert ist, und das zweite Nukleotid (Y) ein reversibler Terminator ist, der mit einer zweiten Blockierungsgruppe blockiert ist.

3. Verfahren nach Anspruch 2, umfassend:
(a) Durchführen von mehreren Zyklen des Folgenden:
(i) weiteres Erweitern der erweiterten Primer unter Verwendung einer ersten Mischung, die eine Polymerase und vier Nukleotidtriphosphate ausgewählt aus A, T, C und G und/oder Analoga davon enthält, wobei eines der Nukleotidtriphosphate oder Analoga in der ersten Mischung dem ersten Nukleotid (X) des ausgewählten Dinukleotids entspricht und mit einer ersten Blockierungsgruppe blockiert ist, und wobei die anderen drei Nukleotidtriphosphate oder Analoga in der ersten Mischung entblockt sind, wobei das Erweitern fortgesetzt wird, bis im Wesentlichen alle der erweiterten Primer mit der ersten Blockierungsgruppe blockiert sind; dann
(ii) Entblocken der ersten Blockierungsgruppe; und
(iii) Behandeln der erweiterten Primer aus (ii) mit einer zweiten Mischung, die eine Polymerase und ein einzelnes Nukleotidtriphosphat ausgewählt aus A, T, C oder G und Analoga davon enthält, das dem zweiten Nukleotid (Y) des ausgewählten Dinukleotids entspricht und mit einer zweiten Blockierungsgruppe blockiert ist, wobei die zweite Mischung optional die drei Nukleotidtriphosphate oder Analoga beinhaltet, die nicht dem zweiten Nukleotid (Y) entsprechen, das mit der ersten Blockierungsgruppe blockiert ist,
(b) Wiederholen von Schritt (a), bis im Wesentlichen alle der erweiterten Primer mit der zweiten Blockierungsgruppe blockiert sind; und
(c) Entblocken der zweiten Blockierungsgruppe;
wodurch die erweiterten Primer in der klonalen Population neu phasiert werden.

4. Verfahren nach Anspruch 3, wobei entweder
(A) das einzige Nukleotidtriphosphat in der zweiten Mischung das Nukleotidtriphosphat oder Analogon ist, das durch die zweite Blockierungsgruppe blockiert ist; oder
(B) die zweite Mischung das Nukleotidtriphosphat oder Analogon, das durch die zweite Gruppe blockiert ist, und die drei Nukleotidtriphosphate oder Analoga, die nicht dem zweiten Nukleotid (Y) entsprechen, das mit der ersten Blockierungsgruppe blockiert ist, enthält.

5. Verfahren nach einem der Ansprüche 2 bis 4, wobei eine von der ersten und der zweiten Blockierungsgruppe eine O-Azidomethylgruppe ist und die andere von der ersten und der zweiten Blockierungsgruppe eine O-NH₂-Gruppe ist.

6. Verfahren nach Anspruch 2, umfassend:
(a) Durchführen von mehreren Zyklen des Folgenden:
(i) weiteres Erweitern der erweiterten Primer unter Verwendung einer ersten Mischung, die eine Polymerase und vier Nukleotidtriphosphate ausgewählt aus A, T, C und G und/oder Analoga davon enthält, wobei eines der Nukleotidtriphosphate oder Analoga in der ersten Mischung dem ersten Nukleotid (X) des ausgewählten Dinukleotids entspricht und mit einer ersten Blockierungsgruppe blockiert ist, und wobei die anderen drei Nukleotidtriphosphate oder Analoga in der ersten Mischung entblockt sind, wobei das Erweitern fortgesetzt wird, bis im Wesentlichen alle der erweiterten Primer mit der ersten Blockierungsgruppe blockiert sind; dann
(ii) Entblocken der ersten Blockierungsgruppe; und
(iii) Behandeln der erweiterten Primer mit einer zweiten Mischung, die eine Ligase und ein 5'-phosphoryliertes Oligonukleotid, das an dem 3'-Ende blockiert ist, enthält, wobei eine Base in dem Oligonukleotid dem zweiten Nukleotid (Y) des ausgewählten Dinukleotids entspricht;
(b) Wiederholen von Schritt (a), bis im Wesentlichen alle der erweiterten Primer mit dem Oligonukleotid blockiert sind; und
(c) Entblocken des Oligonukleotids;
wodurch die erweiterten Primer in der klonalen Population neu phasiert werden.

7. Verfahren nach Anspruch 6, wobei das 5'-phosphorylierte Oligonukleotid die Formel A(N)₁₋₁₅-B aufweist, wobei A eine Nukleotidbase ist, die dem zweiten Nukleotid (Y) des ausgewählten Dinukleotids entspricht, jedes N ein Nukleotidhomologon oder eine Nukleotidmischung ist, die ein Nukleotid enthält, das an eine beliebige Base in der Vorlagensequenz hybridisieren kann; und B eine nicht reversible Blockierungsstruktur ist; und wobei das Entblocken in Schritt (c) Entfernen des Oligonukleotids von dem erweiterten Primer umfasst.

8. Verfahren nach Anspruch 7, wobei die nicht reversible Blockierungsstruktur invertiertes dT (IDT) ist, das an dem 3'-Ende des Oligonukleotids eingebaut ist, wodurch eine 3'-3'-Bindung erzeugt wird, die sowohl Abbau durch 3'-Exonukleasen als auch Erweiterung durch DNA-Polymerasen verhindert.

9. Verfahren nach Anspruch 7, wobei A Uracil ist und wobei 5'-phosphoryliertes Oligonukleotid durch Behandeln mit einer Enzymmischung aus Uracil-DNA-Glycosylase (UDG) und Apurin-/Apyrimidin-Endonuklease 1 (Apel) entblockt wird, um die Uracilbase zu spalten und zu entfernen.

10. Verfahren nach einem der Ansprüche 3 bis 9, wobei fünf bis fünfzehn Zyklen in Schritt (a) durchgeführt werden.

11. Verfahren nach einem der Ansprüche 1 bis 10, wobei fünf bis fünfzig Basen von dem 3'-Ende jedes Primers vor dem Neuphasieren entfernt werden, wodurch das 3'-Ende der erweiterten Primer an eine vorgelagerte Position korrigiert wird.

12. Verfahren nach Anspruch 11, wobei das Korrigieren Folgendes umfasst:
(i) während der Sequenzierung durch Synthese, die vor dem Neuphasieren erfolgt, Einschließen in zumindest einigen der Zyklen der Sequenzierung eines Uraciltriphosphats oder Analogons davon, das in den erweiterten Primer anstelle von Thymintriphosphat eingebaut werden kann; dann
(ii) Spalten der erweiterten Primer an eingebauten Uracilbasen.

13. Verfahren nach Anspruch 12, wobei das Spalten in Schritt (ii) unter Verwendung einer Enzymmischung aus Uracil-DNA-Glycosylase (UDG) und Apurin-/Apyrimidin-Endonuklease 1 (Apel) erfolgt.

14. Verfahren nach Anspruch 11, wobei das Korrigieren Folgendes umfasst:
(i) während der Sequenzierung durch Synthese, die vor dem Neuphasieren erfolgt, Einschließen in zumindest einigen der Zyklen der Sequenzierung eines Nukleotidtriphosphats, das ein Ribonukleotid (RNA) oder ein 5'-Alphaphosphat-Thio-modifiziertes Nukleotid enthält; dann
(ii) Spalten der erweiterten Primer an eingebauten RNA-Basen oder an eingebauten 5'-Alphaphosphat-Thio-modifizierten Nukleotiden.

15. Verfahren nach Anspruch 11, wobei das Korrigieren Behandeln der erweiterten Primer mit einer 3'-Exonuklease unter kontrollierten Bedingungen oder Behandeln der erweiterten Primer mit einem Nicking-Enzym umfasst, das von Subsequenz abhängig ist, wodurch die fünf bis fünfzig Basen von dem 3'-Ende des erweiterten Primers entfernt werden.

16. Verfahren nach einem der Ansprüche 1 bis 15, ferner umfassend Wiederaufnehmen von Zyklen der Sequenzierung nach dem Neuphasieren, wobei die erweiterten Primer in der klonalen Population um Basen erweitert werden, die jeweils ein komplementäres Nukleotid in der Vorlagensequenz identifizieren.

17. Verfahren zum Erhalten von langen Sequenzierungs-Reads aus einer klonalen Population von Nukleinsäureduplexen, die jeweils einen erweiterten Primer umfassen, der an eine Vorlagensequenz angelagert ist, wobei das Verfahren Folgendes umfasst:
Durchführen von mehreren Zyklen von Sequenzierung, in denen der erweiterte Primer in jedem Duplex um ein Nukleotid erweitert wird, wodurch ein komplementäres Nukleotid in der Vorlagensequenz identifiziert wird;
nach einer Anzahl solcher Sequenzierungszyklen Neuphasieren der erweiterten Primer gemäß dem Verfahren nach einem der Ansprüche 1 bis 16; dann
Wiederaufnehmen der Zyklen der Sequenzierung, um weitere Nukleotide in der Vorlagensequenz zu identifizieren.

18. Verfahren nach Anspruch 17, wobei das Neuphasieren zwei- bis viermal innerhalb der ersten 800 Sequenzierungszyklen vorgenommen wird.

19. Verfahren nach Anspruch 17 oder Anspruch 18, wobei das Neuphasieren die Anzahl an klonalen Populationen mit einem Diskordanzprozentsatz von weniger als 2 % um zumindest das 1,5-fache erweitert; oder
wobei das Neuphasieren die Anzahl an klonalen Populationen mit einem Diskordanzprozentsatz von weniger als 2 % um zumindest 200 Zyklen erweitert.

20. Verfahren nach einem der Ansprüche 1-19, wobei jede klonale Population auf dem Array ein DNA-Nanoball oder ein Konkatemer ist; oder
wobei jede klonale Population ein Cluster von DNA-Strängen ist, die durch Brücken-Polymerase-Kettenreaktion (PCR) oder Kopien einer Vorlagensequenz in einem Emulsionströpfchen produziert werden.

## Revendications

1. Méthode de remise en phase d'amorces allongées dans une population clonale de duplexes d'acides nucléiques comprenant des amorces allongées hybridées à une séquence modèle, une pluralité des amorces allongées dans la population clonale comportant différentes extrémités 3' et étant ainsi hors phase, la méthode comprenant :
(1) l'allongement des amorces allongées en incorporant un ou plusieurs nucléotides qui sont complémentaires à la séquence modèle à l'aide d'une polymérase et de nucléotides comprenant les nucléotides triphosphates A, T, C et G, ou leurs analogues, l'un des nucléotides étant un terminateur réversible bloqué par un premier groupe de blocage et les trois autres nucléotides n'étant pas bloqués, jusqu'à ce que sensiblement toutes les amorces allongées soient bloquées ; et ensuite
(2) le déblocage des amorces allongées,
ladite remise en phase comprenant la remise en phase de la fréquence des dinucléotides (DFR), dans laquelle chaque amorce allongée est allongée jusqu'à ce qu'un dinucléotide XY choisi soit obtenu.

2. Méthode de remise en phase selon la revendication 1,
ledit premier nucléotide (X) étant le terminateur réversible bloqué avec le premier groupe de blocage, et ledit second nucléotide (Y) étant le terminateur réversible bloqué avec un second groupe de blocage.

3. Méthode selon la revendication 2 comprenant :
(a) l'exécution de multiples cycles parmi les suivants :
(i) l'allongement supplémentaire des amorces allongées à l'aide d'un premier mélange qui contient une polymérase et quatre nucléotides triphosphates choisis parmi A, T, C et G et/ou leurs analogues, l'un desdits nucléotides triphosphates ou analogues dans le premier mélange correspondant au premier nucléotide (X) du dinucléotide choisi et étant bloqué avec un premier groupe de blocage, et lesdits trois autres nucléotides triphosphates ou analogues dans le premier mélange étant non bloqués, l'allongement se poursuivant jusqu'à ce que sensiblement toutes les amorces allongées soient bloquées avec le premier groupe de blocage ; ensuite
(ii) le déblocage du premier groupe de blocage ; et
(iii) le traitement des amorces allongées provenant de (ii) avec un second mélange qui contient une polymérase et un nucléotide triphosphate unique choisi parmi A, T, C ou G et leurs analogues qui correspond au second nucléotide (Y) du dinucléotide choisi et étant bloqué avec un second groupe de blocage, ledit second mélange comprenant éventuellement les trois nucléotides triphosphates ou analogues ne correspondant pas au second nucléotide (Y) bloqué avec le premier groupe de blocage,
(b) la répétition de l'étape (a) jusqu'à ce que sensiblement toutes les amorces allongées soient bloquées avec le second groupe de blocage ; et
(c) le déblocage du second groupe de blocage ;
d'où la remise en phase des amorces allongées dans la population clonale.

4. Méthode selon la revendication 3, soit
(A) ledit nucléotide triphosphate unique dans le second mélange étant le nucléotide triphosphate ou l'analogue qui est bloqué par le second groupe de blocage ; soit,
(B) ledit second mélange contenant le nucléotide triphosphate ou l'analogue bloqué par le second groupe, et lesdits trois nucléotides triphosphates ou analogues ne correspondant pas au second nucléotide (Y) bloqué avec le premier groupe de blocage.

5. Méthode selon l'une quelconque des revendications 2 à 4, l'un des premier et second groupes de blocage étant un groupe O-azidométhyle, et l'autre des premier et second groupes de blocage étant un groupe O-NH₂.

6. Méthode selon la revendication 2 comprenant :
(a) l'exécution de multiples cycles parmi les suivants :
(i) l'allongement supplémentaire des amorces allongées à l'aide d'un premier mélange qui contient une polymérase et quatre nucléotides triphosphates choisis parmi A, T, C et G et/ou leurs analogues, l'un desdits nucléotides triphosphates ou analogues dans le premier mélange correspondant au premier nucléotide (X) du dinucléotide choisi et étant bloqué avec un premier groupe de blocage, et lesdits trois autres nucléotides triphosphates ou analogues dans le premier mélange étant non bloqués, l'allongement se poursuivant jusqu'à ce que sensiblement toutes les amorces allongées soient bloquées avec le premier groupe de blocage ; ensuite
(ii) le déblocage du premier groupe de blocage ; et
(iii) le traitement des amorces allongées avec un second mélange qui contient une ligase et un oligonucléotide phosphorylé 5' bloqué au niveau de l'extrémité 3', une base dans l'oligonucléotide correspondant au second nucléotide (Y) du dinucléotide choisi ;
(b) la répétition de l'étape (a) jusqu'à ce que sensiblement toutes les amorces allongées soient bloquées avec le second groupe de blocage ; et
(c) le déblocage de l'oligonucléotide ;
d'où la remise en phase des amorces allongées dans la population clonale.

7. Méthode selon la revendication 6, ledit oligonucléotide phosphorylé 5' comportant la formule A(N)₁₋₁₅-B, dans laquelle A représente une base nucléotidique qui correspond au second nucléotide (Y) du dinucléotide choisi, chaque N représente un homologue nucléotidique ou un mélange nucléotidique contenant un nucléotide qui peut s'hybrider à une quelconque base dans la séquence modèle ; et B représente une structure de blocage non réversible ; et ledit déblocage à l'étape (c) comprenant le retrait de l'oligonucléotide de l'amorce allongée.

8. Méthode selon la revendication 7, ladite structure de blocage non réversible étant un dT inversé (IDT) incorporé au niveau de l'extrémité 3' de l'oligonucléotide, ce qui permet la création d'une liaison 3'-3' qui inhibe à la fois la dégradation par les exonucléases 3' et l'extension par les ADN polymérases.

9. Méthode selon la revendication 7, A étant un uracile, et ledit oligonucléotide phosphorylé 5' étant débloqué par traitement avec un mélange enzymatique d'uracile-ADN glycosylase (UDG) et d'endonucléase 1 apurinique/apyrimidinique (Ape1) pour cliver et retirer la base uracile.

10. Méthode selon l'une quelconque des revendications 3 à 9, cinq à quinze cycles étant exécutés à l'étape (a).

11. Méthode selon l'une quelconque des revendications 1 à 10, cinq à cinquante bases étant retirées de l'extrémité 3' de chaque amorce avant la remise en phase, ce qui permet le réajustement de l'extrémité 3' des amorces allongées vers une position en amont.

12. Méthode selon la revendication 11, ledit réajustement comprenant :
(i) pendant le séquençage par synthèse effectué avant la remise en phase, l'inclusion dans un moins certains des cycles du séquençage d'un uracile triphosphate ou analogue de celui-ci qui peut être incorporé dans l'amorce allongée à la place d'une thymine triphosphate ; ensuite
(ii) le clivage des amorces allongées au niveau des bases uracile incorporées.

13. Méthode selon la revendication 12, ledit clivage à l'étape (ii) étant effectué à l'aide d'un mélange enzymatique d'uracile-ADN glycosylase (UDG) et d'endonucléase 1 apurinique/apyrimidinique (Ape1).

14. Méthode selon la revendication 11, ledit réajustement comprenant :
(i) pendant le séquençage par synthèse effectué avant la remise en phase, l'inclusion dans un moins certains des cycles du séquençage d'un nucléotide triphosphate qui contient un ribonucléotide (ARN) ou d'un alpha-phosphate nucléotide 5' thio-modifié ; ensuite
(ii) le clivage des amorces allongées au niveau des bases d'ARN incorporées ou au niveau des alpha-phosphate nucléotides 5' thio-modifiés incorporés.

15. Méthode selon la revendication 11, ledit réajustement comprenant le traitement des amorces allongées avec une exonucléase 3' dans des conditions contrôlées, ou le traitement des amorces allongées avec une enzyme de coupure qui dépend d'une sous-séquence, ce qui permet le retrait desdites cinq à cinquante bases de l'extrémité 3' de l'amorce allongée.

16. Méthode selon l'une quelconque des revendications 1 à 15, comprenant en outre la reprise des cycles de séquençage après la remise en phase, grâce à quoi les amorces dans la population clonale sont allongées par des bases qui identifient chacune un nucléotide complémentaire dans la séquence modèle.

17. Méthode d'obtention de longues lectures de séquençage à partir d'une population clonale de duplexes d'acides nucléiques comprenant chacun une amorce allongée annelée à une séquence modèle, la méthode comprenant :
la réalisation de multiples cycles de séquençage dans lesquels l'amorce allongée dans chaque duplex est allongée par un nucléotide, ce qui permet l'identification d'un nucléotide complémentaire dans la séquence modèle ;
après un certain nombre de de ces cycles de séquençage, la remise en phase des amorces allongées conformément à la méthode selon les revendications 1 à 16 ; ensuite
la reprise des cycles de séquençage pour identifier les nucléotides supplémentaires dans la séquence modèle.

18. Méthode selon la revendication 17, ladite remise en phase étant effectuée deux à quatre fois durant les 800 premiers cycles de séquençage.

19. Méthode selon la revendication 17 ou la revendication 18, ladite remise en phase augmentant le nombre de populations clonales comportant un pourcentage de divergence inférieur à 2 % d'un facteur d'au moins 1,5 ; ou,
ladite remise en phase augmentant le nombre de populations clonales comportant un pourcentage de divergence inférieur à 2 % d'au moins 200 cycles.

20. Méthode selon l'une quelconque des revendications 1 à 19, chaque population clonale sur la matrice étant une nanobille ou un concatémère d'ADN ; ou
chaque population clonale étant un groupe de brins d'ADN produit par réaction en chaîne par polymérase (PCR) en pont ou des copies d'une séquence modèle dans une gouttelette d'émulsion.
